# EUROPEAN PATENT APPLICATION

(11) **EP 1 136 554 A1**
(43) Date of publication of application: **26.09.2001**
(21) Application number: 00106401.3
(22) Date of filing: 24.03.2000
(51) Int. Cl.: C12N 15/12, C12N 15/11, C12N 9/00, C12N 5/10, C07K 14/705, C07K 16/28, C12Q 1/68, G01N 33/53, A61K 48/00

(54) **ATP binding cassette transporter 1 (ABC1) gene polymorphisms and uses thereof for the diagnosis and treatment of lipid disorders, cardiovascular diseases and inflammatory diseases**

(71) Applicant: Bayer Aktiengesellschaft, 51368 Leverkusen (DE)
(72) Inventor: Schmitz, Gerd, Prof.Dr., 93161 Sinzig (DE); Bodzioch, Marek, Dr., 30-601 Krakow (PL)

(57) **Abstract**

The present invention provides four common polymorphisms in the gene encoding the ATP-binding cassette transporter-1 (*ABC1*), which are associated with decreased ApoA-I mediated efflux of cholesterol, the first step of reverse cholesterol transport. The provided data can be taken as evidence that common polymorphisms in *ABC1* directly affect cellular lipid homeostasis, which is a key factor in the atherogenetic process. The frequencies of three of the common *ABC1* variants are significantly increased in a population of men having low HDL-C levels and established CHD relative to CHD-free control subjects. The use of the provided ABC1 polymorphisms for the diagnosis and treatment of lipid disorders, cardiovascular diseases, and inflammatory diseases like psoriasis and lupus erythematodes is claimed.

SEQUENCE LISTING

<110> Bayer AG

<120> ATP binding cassette transporter 1 (ABC1) gene
      polymorphisms and uses thereof for the diagnosis and
      treatment of lipid disorders, cardiovascular diseases
      and inflammatory diseases

<130> abc transporter diagnostic

<140>
<141>

<160> 4

<170> PatentIn Ver. 2.1

<210> 1
<211> 6880
<212> DNA
<213> Human

<400> 1
caaacatgtc agctgttact ggaagtggcc tggcctctat ttatcttcct gatcctgatc 60
tctgttcggc tgagctaccc accctatgaa caacatgaat gccattttcc aaataaagcc 120
atgccctctg caggaacact tccttgggtt caggggatta tctgtaatgc caacaacccc 180
tgtttccgtt acccgactcc tggggaggct cccggagttg ttggaaactt taacaaatcc 240
attgtggctc gcctgttctc agatgctcgg aggcttcttt tatacagcca gaaagacacc 300
agcatgaagg acatgcgcaa agttctgaga acattacagc agatcaagaa atccagctca 360
aacttgaagc ttcaagattt cctggtggac aatgaaacct tctctgggtt cctgtatcac 420
aacctctctc tcccaaagtc tactgtggac aagatgctga gggctgatgt cattctccac 480
aaggtatttt tgcaaggcta ccagttacat ttgacaagtc tgtgcaatgg atcaaaatca 540
gaagagatga ttcaacttgg tgaccaagaa gtttctgagc tttgtggcct accaagggag 600
aaactggctg cagcagagcg agtacttcgt tccaacatgg acatcctgaa gccaatcctg 660
agaacactaa actctacatc tcccttcccg agcaaggagc tggccgaagc cacaaaaaca 720
ttgctgcata gtcttgggac tctggcccag gagctgttca gcatgagaag ctggagtgac 780
atgcgacagg aggtgatgtt tctgaccaat gtgaacagct ccagctcctc cacccaaatc 840
taccaggctg tgtctcgtat tgtctgcggg catcccgagg gaggggggct gaagatcaag 900
tctctcaact ggtatgagga caacaactac aaagccctct ttggaggcaa tggcactgag 960
gaagatgctg aaaccttcta tgacaactct acaactcctt actgcaatga tttgatgaag 1020
aatttggagt ctagtcctct ttcccgcatt atctggaaag ctctgaagcc gctgctcgtt 1080
gggaagatcc tgtatacacc tgacactcca gccacaaggc aggtcatggc tgaggtgaac 1140
aagaccttcc aggaactggc tgtgttccat gatctggaag gcatgtggga ggaactcagc 1200
cccaagatct ggaccttcat ggagaacagc caagaaatgg accttgtccg gatgctgttg 1260
gacagcaggg acaatgacca cttttgggaa cagcagttgg atggcttaga ttggacagcc 1320
caagacatcg tggcgttttt ggccaagcac ccagaggatg tccagtccag taatggttct 1380
gtgtacacct ggagagaagc tttcaacgag actaaccagg caatccggac catatctcgc 1440
ttcatggagt gtgtcaacct gaacaagcta gaacccatag caacagaagt ctggctcatc 1500
aacaagtcca tggagctgct ggatgagagg aagttctggg ctggtattgt gttcactgga 1560
attactccag gcagcattga gctgccccat catgtcaagt acaagatccg aatggacatt 1620
gacaatgtgg agaggacaaa taaaatcaag gatgggtact gggaccctgg tcctcgagct 1680
gacccctttg aggacatgcg gtacgtctgg gggggcttcg cctacttgca ggatgtggtg 1740
gagcaggcaa tcatcagggt gctgacgggc accgagaaga aaactggtgt ctatatgcaa 1800
cagatgccct atccctgtta cgttgatgac atctttctgc gggtgatgag ccggtcaatg 1860
cccctcttca tgacgctggc ctggatttac tcagtggctg tgatcatcaa gggcatcgtg 1920
tatgagaagg aggcacggct gaaagagacc atgcggatca tgggcctgga caacagcatc 1980
ctctggttta gctggttcat tagtagcctc attcctcttc ttgtgagcgc tggcctgcta 2040
gtggtcatcc tgaagttagg aaacctgctg ccctacagtg atcccagcgt ggtgtttgtc 2100
ttcctgtccg tgtttgctgt ggtgacaatc ctgcagtgct tcctgattag cacactcttc 2160
tccagagcca acctggcagc agcctgtggg ggcatcatct acttcacgct gtacctgccc 2220
tacgtcctgt gtgtggcatg gcaggactac gtgggcttca cactcaagat cttcgctagc 2280
ctgctgtctc ctgtggcttt tgggtttggc tgtgagtact ttgccctttt tgaggagcag 2340

ggcattggag tgcagtggga caacctgttt gagagtcctg tggaggaaga tggcttcaat 2400
ctcaccactt cggtctccat gatgctgttt gacaccttcc tctatggggt gatgacctgg 2460
tacattgagg ctgtctttcc aggccagtac ggaattccca ggccctggta ttttccttgc 2520
accaagtcct actggtttgg cgaggaaagt gatgagaaga gccaccctgg ttccaaccag 2580
aagagaatat cagaaatctg catggaggag gaacccaccc acttgaagct gggcgtgtcc 2640
attcagaacc tggtaaaagt ctaccgagat gggatgaagg tggctgtcga tggcctggca 2700
ctgaattttt atgaggccca gatcacctcc ttcctgggcc acaatggagc ggggaagacg 2760
accaccatgt caatcctgac cgggttgttc cccccgacct cgggcaccgc ctacatcctg 2820
ggaaaagaca ttcgctctga gatgagcacc atccggcaga acctgggggt ctgtccccag 2880
cataacgtgc tgtttgacat gctgactgtc gaagaacaca tctggttcta tgcccgcttg 2940
aaagggctct ctgagaagca cgtgaaggcg gagatggagc agatggccct ggatgttggt 3000
ttgccatcaa gcaagctgaa aagcaaaaca agccagctgt caggtggaat gcagagaaag 3060
ctatctgtgg ccttggcctt tgtcggggga tctaaggttg tcattctgga tgaacccaca 3120
gctggtgtgg acccttactc ccgcagggga atatgggagc tgctgctgaa ataccgacaa 3180
ggccgcacca ttattctctc tacacaccac atggatgaag cggacgtcct gggggacagg 3240
attgccatca tctcccatgg gaagctgtgc tgtgtgggct cctccctgtt tctgaagaac 3300
cagctgggaa caggctacta cctgaccttg gtcaagaaag atgtggaatc ctccctcagt 3360
tcctgcagaa acagtagtag cactgtgtca tacctgaaaa aggaggacag tgtttctcag 3420
agcagttctg atgctggcct gggcagcgac catgagagtg acacgctgac catcgatgtc 3480
tctgctatct ccaacctcat caggaagcat gtgtctgaag cccggctggt ggaagacata 3540
gggcatgagc tgacctatgt gctgccatat gaagctgcta aggagggagc ctttgtggaa 3600
ctctttcatg agattgatga ccggctctca gacctgggca tttctagtta tggcatctca 3660
gagacgaccc tggaagaaat attcctcaag gtggccgaag agagtggggt ggatgctgag 3720
acctcagatg gtaccttgcc agcaagacga aacaggcggg ccttcgggga caagcagagc 3780
tgtcttcgcc cgttcactga agatgatgct gctgatccaa atgattctga catagaccca 3840
gaatccagag agacagactt gctcagtggg atggatggca aagggtccta ccaggtgaaa 3900
ggctggaaac ttacacagca acagtttgtg gcccttttgt ggaagagact gctaattgcc 3960
agacggagtc ggaaaggatt ttttgctcag attgtcttgc cagctgtgtt tgtctgcatt 4020
gcccttgtgt tcagcctgat cgtgccaccc tttggcaagt accccagcct ggaacttcag 4080
ccctggatgt acaacgaaca gtacacattt gtcagcaatg atgctcctga ggacacggga 4140
accctggaac tcttaaacgc cctcaccaaa gaccctggct tcgggacccg ctgtatggaa 4200
ggaaacccaa tcccagacac gccctgccag gcaggggagg aagagtggac cactgcccca 4260
gttccccaga ccatcatgga cctcttccag aatgggaact ggacaatgca gaacccttca 4320
cctgcatgcc agtgtagcag cgacaaaatc aagaagatgc tgcctgtgtg tcccccaggg 4380
gcaggggggc tgcctcctcc acaaagaaaa caaaacactg cagatatcct tcaggacctg 4440
acaggaagaa acatttcgga ttatctggtg aagacgtatg tgcagatcat agccaaaagc 4500
ttaaagaaca agatctgggt gaatgagttt aggtatggcg gcttttccct gggtgtcagt 4560
aatactcaag cacttcctcc gagtcaagaa gttaatgatg ccaccaaaca aatgaagaaa 4620
cacctaaagc tggccaagga cagttctgca gatcgatttc tcaacagctt gggaagattt 4680
atgacaggac tggacaccag aaataatgtc aaggtgtggt tcaataacaa gggctggcat 4740
gcaatcagct ctttcctgaa tgtcatcaac aatgccattc tccgggccaa cctgcaaaag 4800
ggagagaacc ctagccatta tggaattact gctttcaatc atcccctgaa tctcaccaag 4860
cagcagctct cagaggtggc tccgatgacc acatcagtgg atgtccttgt gtccatctgt 4920
gtcatctttg caatgtcctt cgtcccagcc agctttgtcg tattcctgat ccaggagcgg 4980
gtcagcaaag caaaacacct gcagttcatc agtggagtga agcctgtcat ctactggctc 5040
tctaattttg tctgggatat gtgcaattac gttgtccctg ccacactggt cattatcatc 5100
ttcatctgct tccagcagaa gtcctatgtg tcctccacca atctgcctgt gctagccctt 5160
ctacttttgc tgtatgggtg gtcaatcaca cctctcatgt acccagcctc ctttgtgttc 5220
aagatcccca gcacagccta tgtggtgctc accagcgtga acctcttcat tggcattaat 5280
ggcagcgtgg ccacctttgt gctggagctg ttcaccgaca ataagctgaa taatatcaat 5340
gatatcctga agtccgtgtt cttgatcttc ccacattttt gcctgggacg agggctcatc 5400
gacatggtga aaaaccaggc aatggctgat gccctggaaa ggtttgggga gaatcgcttt 5460
gtgtcaccat tatcttggga cttggtggga cgaaacctct tcgccatggc cgtggaaggg 5520
gtggtgttct tcctcattac tgttctgatc cagtacagat tcttcatcag gcccagacct 5580
gtaaatgcaa agctatctcc tctgaatgat gaagatgaag atgtgaggcg ggaaagacag 5640
agaattcttg atggtggagg ccagaatgac atcttagaaa tcaaggagtt gacgaagata 5700
tatagaagga agcggaagcc tgctgttgac aggatttgcg tgggcattcc tcctggtgag 5760
tgctttgggc tcctgggagt taatggggct ggaaaatcat caactttcaa gatgttaaca 5820
ggagatacca ctgttaccag aggagatgct ttccttaaca gaaatagtat cttatcaaac 5880
atccatgaag tacatcagaa catgggctac tgccctcagt ttgatgccat cacagagctg 5940
ttgactggga gagaacacgt ggagttcttt gcccttttga gaggagtccc agagaaagaa 6000
gttggcaagg ttggtgagtg ggcgattcgg aaactgggcc tcgtgaagta tggagaaaaa 6060
tatgctggta actatagtgg aggcaacaaa cgcaagctct ctacagccat ggctttgatc 6120

ggcgggcctc ctgtggtgtt tctggatgaa cccaccacag gcatggatcc caaagcccgg 6180
cggttcttgt ggaattgtgc cctaagtgtt gtcaaggagg ggagatcagt agtgcttaca 6240
tctcatagta tggaagaatg tgaagctctt tgcactagga tggcaatcat ggtcaatgga 6300
aggttcaggt gccttggcag tgtccagcat ctaaaaaata ggtttggaga tggttataca 6360
atagttgtac gaatagcagg gtccaacccg gacctgaagc ctgtccagga tttctttgga 6420
cttgcatttc ctggaagtgt tccaaaagag aaacaccgga acatgctaca ataccagctt 6480
ccatcttcat tatcttctct ggccaggata ttcagcatcc tctcccagag caaaaagcga 6540
ctccacatag aagactactc tgtttctcag acaacacttg accaagtatt tgtgaacttt 6600
gccaaggacc aaagtgatga tgaccactta aaagacctct cattacacaa aaaccagaca 6660
gtagtggacg ttgcagttct cacatctttt ctacaggatg agaaagtgaa agaaagctat 6720
gtatgaagaa tcctgttcat acggggtggc tgaaagtaaa gagggactag actttccttt 6780
gcaccatgtg aagtgttgtg gagaaaagag ccagaagttg atgtgggaag aagtaaactg 6840
gatactgtac tgatactatt caatgcaatg caattcaatg 6880

<210> 2
<211> 2201
<212> PRT
<213> Human

<400> 2

<210> 3
<211> 7260
<212> DNA
<213> Human

<400> 3
aaaccccgta attgcgagcg agagtgagtg gggccgggac ccgcagagcc gagccgaccc 60
ttctctcccg ggctgcggca gggcagggcg gggagctccg cgcaccaaca gagccggttc 120
tcagggcgct ttgctccttg ttttttcccc ggttctgttt tctccccttc tccggaaggc 180
ttgtcaaggg gtaggagaaa gagacgcaaa cacaaaagtg gaaaacagtt aatgaccagc 240
cacggcgtcc ctgctgtgag ctctggccgc tgccttccag ggctcccgag ccacacgctg 300
ggggtgctgg ctgagggaac atggcttgtt ggcctcagct gaggttgctg ctgtggaaga 360
acctcacttt cagaagaaga caaacatgtc agctgttact ggaagtggcc tggcctctat 420
ttatcttcct gatcctgatc tctgttcggc tgagctaccc accctatgaa caacatgaat 480
gccattttcc aaataaagcc atgccctctg caggaacact tccttgggtt caggggatta 540
tctgtaatgc caacaacccc tgtttccgtt acccgactcc tggggaggct cccggagttg 600
ttggaaactt taacaaatcc attgtggctc gcctgttctc agatgctcgg aggcttcttt 660
tatacagcca gaaagacacc agcatgaagg acatgcgcaa agttctgaga acattacagc 720
agatcaagaa atccagctca aacttgaagc ttcaagattt cctggtggac aatgaaacct 780
tctctgggtt cctgtatcac aacctctctc tcccaaagtc tactgtggac aagatgctga 840
gggctgatgt cattctccac aaggtatttt tgcaaggcta ccagttacat ttgacaagtc 900
tgtgcaatgg atcaaaatca gaagagatga ttcaacttgg tgaccaagaa gtttctgagc 960
tttgtggcct accaaggagg aaactggctg cagcagagcg agtacttcgt tccaacatgg 1020
acatcctgaa gccaatcctg agaacactaa actctacatc tcccttcccg agcaaggagc 1080
tggccgaagc cacaaaaaca ttgctgcata gtcttgggac tctggcccag gagctgttca 1140
gcatgagaag ctggagtgac atgcgacagg aggtgatgtt tctgaccaat gtgaacagct 1200
ccagctcctc cacccaaatc taccaggctg tgtctcgtat tgtctgcggg catcccgagg 1260
gaggggggct gaagatcaag tctctcaact ggtatgagga caacaactac aaagccctct 1320
ttggaggcaa tggcactgag gaagatgctg aaaccttcta tgacaactct acaactcctt 1380
actgcaatga tttgatgaag aatttggagt ctagtcctct ttcccgcatt atctggaaag 1440
ctctgaagcc gctgctcgtt gggaagatcc tgtatacacc tgacactcca gccacaaggc 1500
aggtcatggc tgaggtgaac aagaccttcc aggaactggc tgtgttccat gatctggaag 1560
gcatgtggga ggaactcagc cccaagatct ggaccttcat ggagaacagc caagaaatgg 1620
accttgtccg gatgctgttg gacagcaggg acaatgacca cttttgggaa cagcagttgg 1680
atggcttaga ttggacagcc caagacatcg tggcgttttt ggccaagcac ccagaggatg 1740
tccagtccag taatggttct gtgtacacct ggagagaagc tttcaacgag actaaccagg 1800
caatccggac catatctcgc ttcatggagt gtgtcaacct gaacaagcta gaacccatag 1860
caacagaagt ctggctcatc aacaagtcca tggagctgct ggatgagagg aagttctggg 1920
ctggtattgt gttcactgga attactccag gcagcattga gctgccccat catgtcaagt 1980
acaagatccg aatggacatt gacaatgtgg agaggacaaa taaaatcaag gatgggtact 2040
gggaccctgg tcctcgagct gacccctttg aggacatgcg gtacgtctgg gggggcttcg 2100
cctacttgca ggatgtggtg gagcaggcaa tcatcagggt gctgacgggc accgagaaga 2160
aaactggtgt ctatatgcaa cagatgccct atccctgtta cgttgatgac atctttctgc 2220
gggtgatgag ccggtcaatg cccctcttca tgacgctggc ctggatttac tcagtggctg 2280
tgatcatcaa gggcatcgtg tatgagaagg aggcacggct gaaagagacc atgcggatca 2340
tgggcctgga caacagcatc ctctggttta gctggttcat tagtagcctc attcctcttc 2400
ttgtgagcgc tggcctgcta gtggtcatcc tgaagttagg aaacctgctg ccctacagtg 2460
atcccagcgt ggtgtttgtc ttcctgtccg tgtttgctgt ggtgacaatc ctgcagtgct 2520
tcctgattag cacactcttc tccagagcca acctggcagc agcctgtggg ggcatcatct 2580
acttcacgct gtacctgccc tacgtcctgt gtgtggcatg gcaggactac gtgggcttca 2640
cactcaagat cttcgctagc ctgctgtctc ctgtggcttt tgggtttggc tgtgagtact 2700
ttgccctttt tgaggagcag ggcattggag tgcagtggga caacctgttt gagagtcctg 2760
tggaggaaga tggcttcaat ctcaccactt cggtctccat gatgctgttt gacaccttcc 2820
tctatggggt gatgacctgg tacattgagg ctgtctttcc aggccagtac ggaattccca 2880
ggccctggta ttttccttgc accaagtcct actggtttgg cgaggaaagt gatgagaaga 2940
gccaccctgg ttccaaccag aagagaatat cagaaatctg catggaggag gaacccaccc 3000
acttgaagct gggcgtgtcc attcagaacc tggtaaaagt ctaccgagat gggatgaagg 3060
tggctgtcga tggcctggca ctgaattttt atgagggcca gatcacctcc ttcctgggcc 3120
acaatggagc ggggaagacg accaccatgt caatcctgac cgggttgttc cccccgacct 3180
cgggcaccgc ctacatcctg ggaaaagaca ttcgctctga gatgagcacc atccggcaga 3240
acctgggggt ctgtccccag cataacgtgc tgtttgacat gctgactgtc gaagaacaca 3300
tctggttcta tgcccgcttg aaagggctct ctgagaagca cgtgaaggcg gagatggagc 3360
agatggccct ggatgttggt ttgccatcaa gcaagctgaa aagcaaaaca agccagctgt 3420

caggtggaat gcagagaaag ctatctgtgg ccttggcctt tgtcggggga tctaaggttg 3480
tcattctgga tgaacccaca gctggtgtgg acccttactc ccgcagggga atatgggagc 3540
tgctgctgaa ataccgacaa ggccgcacca ttattctctc tacacaccac atggatgaag 3600
cggacgtcct gggggacagg attgccatca tctcccatgg gaagctgtgc tgtgtgggct 3660
cctccctgtt tctgaagaac cagctgggaa caggctacta cctgaccttg gtcaagaaag 3720
atgtggaatc ctccctcagt tcctgcagaa acagtagtag cactgtgtca tacctgaaaa 3780
aggaggacag tgtttctcag agcagttctg atgctggcct gggcagcgac catgagagtg 3840
acacgctgac catcgatgtc tctgctatct ccaacctcat caggaagcat gtgtctgaag 3900
cccggctggt ggaagacata gggcatgagc tgacctatgt gctgccatat gaagctgcta 3960
aggagggagc ctttgtggaa ctctttcatg agattgatga ccggctctca gacctgggca 4020
tttctagtta tggcatctca gagacgaccc tggaagaaat attcctcaag gtggccgaag 4080
agagtggggt ggatgctgag acctcagatg gtaccttgcc agcaagacga aacaggcggg 4140
ccttcgggga caagcagagc tgtcttcgcc cgttcactga agatgatgct gctgatccaa 4200
atgattctga catagaccca gaatccagag agacagactt gctcagtggg atggatggca 4260
aagggtccta ccaggtgaaa ggctggaaac ttacacagca acagtttgtg gcccttttgt 4320
ggaagagact gctaattgcc agacggagtc ggaaaggatt ttttgctcag attgtcttgc 4380
cagctgtgtt tgtctgcatt gcccttgtgt tcagcctgat cgtgccaccc tttggcaagt 4440
accccagcct ggaacttcag ccctggatgt acaacgaaca gtacacattt gtcagcaatg 4500
atgctcctga ggacacggga accctggaac tcttaaacgc cctcaccaaa gaccctggct 4560
tcgggacccg ctgtatggaa ggaaacccaa tcccagacac gccctgccag gcaggggagg 4620
aagagtggac cactgcccca gttccccaga ccatcatgga cctcttccag aatgggaact 4680
ggacaatgca gaacccttca cctgcatgcc agtgtagcag cgacaaaatc aagaagatgc 4740
tgcctgtgtg tcccccaggg gcaggggggc tgcctcctcc acaaagaaaa caaaacactg 4800
cagatatcct tcaggacctg acaggaagaa acatttcgga ttatctggtg aagacgtatg 4860
tgcagatcat agccaaaagc ttaaagaaca agatctgggt gaatgagttt aggtatggcg 4920
gcttttccct gggtgtcagt aatactcaag cacttcctcc gagtcaagaa gttaatgatg 4980
ccaccaaaca aatgaagaaa cacctaaagc tggccaagga cagttctgca gatcgatttc 5040
tcaacagctt gggaagattt atgacaggac tggacaccag aaataatgtc aaggtgtggt 5100
tcaataacaa gggctggcat gcaatcagct ctttcctgaa tgtcatcaac aatgccattc 5160
tccgggccaa cctgcaaaag ggagagaacc ctagccatta tggaattact gctttcaatc 5220
atcccctgaa tctcaccaag cagcagctct cagaggtggc tccgatgacc acatcagtgg 5280
atgtccttgt gtccatctgt gtcatctttg caatgtcctt cgtcccagcc agctttgtcg 5340
tattcctgat ccaggagcgg gtcagcaaag caaaacacct gcagttcatc agtggagtga 5400
agcctgtcat ctactggctc tctaattttg tctgggatat gtgcaattac gttgtccctg 5460
ccacactggt cattatcatc ttcatctgct tccagcagaa gtcctatgtg tcctccacca 5520
atctgcctgt gctagccctt ctacttttgc tgtatgggtg gtcaatcaca cctctcatgt 5580
acccagcctc ctttgtgttc aagatcccca gcacagccta tgtggtgctc accagcgtga 5640
acctcttcat tggcattaat ggcagcgtgg ccacctttgt gctggagctg ttcaccgaca 5700
ataagctgaa taatatcaat gatatcctga agtccgtgtt cttgatcttc ccacattttt 5760
gcctgggacg agggctcatc gacatggtga aaaaccaggc aatggctgat gccctggaaa 5820
ggtttgggga gaatcgcttt gtgtcaccat tatcttggga cttggtggga cgaaacctct 5880
tcgccatggc cgtggaaggg gtggtgttct tcctcattac tgttctgatc cagtacagat 5940
tcttcatcag gcccagacct gtaaatgcaa agctatctcc tctgaatgat gaagatgaag 6000
atgtgaggcg ggaaagacag agaattcttg atggtggagg ccagaatgac atcttagaaa 6060
tcaaggagtt gacgaagata tatagaagga agcggaagcc tgctgttgac aggatttgcg 6120
tgggcattcc tcctggtgag tgctttgggc tcctgggagt taatggggct ggaaaatcat 6180
caactttcaa gatgttaaca ggagatacca ctgttaccag aggagatgct ttccttaaca 6240
gaaatagtat cttatcaaac atccatgaag tacatcagaa catgggctac tgccctcagt 6300
ttgatgccat cacagagctg ttgactggga gagaacacgt ggagttcttt gcccttttga 6360
gaggagtccc agagaaagaa gttggcaagg ttggtgagtg ggcgattcgg aaactgggcc 6420
tcgtgaagta tggagaaaaa tatgctggta actatagtgg aggcaacaaa cgcaagctct 6480
ctacagccat ggctttgatc ggcgggcctc ctgtggtgtt tctggatgaa cccaccacag 6540
gcatggatcc caaagcccgg cggttcttgt ggaattgtgc cctaagtgtt gtcaaggagg 6600
ggagatcagt agtgcttaca tctcatagta tggaagaatg tgaagctctt tgcactagga 6660
tggcaatcat ggtcaatgga aggttcaggt gccttggcag tgtccagcat ctaaaaaata 6720
ggtttggaga tggttataca atagttgtac gaatagcagg gtccaacccg gacctgaagc 6780
ctgtccagga tttctttgga cttgcatttc ctggaagtgt tccaaaagag aaacaccgga 6840
acatgctaca ataccagctt ccatcttcat tatcttctct ggccaggata ttcagcatcc 6900
tctcccagag caaaaagcga ctccacatag aagactactc tgtttctcag acaacacttg 6960
accaagtatt tgtgaacttt gccaaggacc aaagtgatga tgaccactta aaagacctct 7020
cattacacaa aaaccagaca gtagtggacg ttgcagttct cacatctttt ctacaggatg 7080
agaaagtgaa agaaagctat gtatgaagaa tcctgttcat acggggtggc tgaaagtaaa 7140
gagggactag actttccttt gcaccatgtg aagtgttgtg gagaaaagag ccagaagttg 7200

atgtgggaag aagtaaactg gatactgtac tgatactatt caatgcaatg caattcaatg 7260

<210> 4
<211> 2261
<212> PRT
<213> Human

<400> 4
Met Ala Cys Trp Pro Gln Leu Arg Leu Leu Leu Trp Lys Asn Leu Thr
1               5                   10                  15

Phe Arg Arg Arg Gln Thr Cys Gln Leu Leu Leu Glu Val Ala Trp Pro
            20                  25                  30

Leu Phe Ile Phe Leu Ile Leu Ile Ser Val Arg Leu Ser Tyr Pro Pro
        35                  40                  45

Tyr Glu Gln His Glu Cys His Phe Pro Asn Lys Ala Met Pro Ser Ala
    50                  55                  60

Gly Thr Leu Pro Trp Val Gln Gly Ile Ile Cys Asn Ala Asn Asn Pro
65                  70                  75                  80

Cys Phe Arg Tyr Pro Thr Pro Gly Glu Ala Pro Gly Val Val Gly Asn
                85                  90                  95

Phe Asn Lys Ser Ile Val Ala Arg Leu Phe Ser Asp Ala Arg Arg Leu
            100                 105                 110

Leu Leu Tyr Ser Gln Lys Asp Thr Ser Met Lys Asp Met Arg Lys Val
        115                 120                 125

Leu Arg Thr Leu Gln Gln Ile Lys Lys Ser Ser Ser Asn Leu Lys Leu
    130                 135                 140

Gln Asp Phe Leu Val Asp Asn Glu Thr Phe Ser Gly Phe Leu Tyr His
145                 150                 155                 160

Asn Leu Ser Leu Pro Lys Ser Thr Val Asp Lys Met Leu Arg Ala Asp
                165                 170                 175

Val Ile Leu His Lys Val Phe Leu Gln Gly Tyr Gln Leu His Leu Thr
            180                 185                 190

Ser Leu Cys Asn Gly Ser Lys Ser Glu Glu Met Ile Gln Leu Gly Asp
        195                 200                 205

Gln Glu Val Ser Glu Leu Cys Gly Leu Pro Arg Glu Lys Leu Ala Ala
    210                 215                 220

Ala Glu Arg Val Leu Arg Ser Asn Met Asp Ile Leu Lys Pro Ile Leu
225                 230                 235                 240

Arg Thr Leu Asn Ser Thr Ser Pro Phe Pro Ser Lys Glu Leu Ala Glu
                245                 250                 255

Ala Thr Lys Thr Leu Leu His Ser Leu Gly Thr Leu Ala Gln Glu Leu
            260                 265                 270

Phe Ser Met Arg Ser Trp Ser Asp Met Arg Gln Glu Val Met Phe Leu
        275                 280                 285

Thr Asn Val Asn Ser Ser Ser Ser Ser Thr Gln Ile Tyr Gln Ala Val
    290                 295                 300

Ser Arg Ile Val Cys Gly His Pro Glu Gly Gly Gly Leu Lys Ile Lys
305                 310                 315                 320

Ser Leu Asn Trp Tyr Glu Asp Asn Asn Tyr Lys Ala Leu Phe Gly Gly
                325                 330                 335

Asn Gly Thr Glu Glu Asp Ala Glu Thr Phe Tyr Asp Asn Ser Thr Thr
            340                 345                 350

Pro Tyr Cys Asn Asp Leu Met Lys Asn Leu Glu Ser Ser Pro Leu Ser
        355                 360                 365

Arg Ile Ile Trp Lys Ala Leu Lys Pro Leu Leu Val Gly Lys Ile Leu
    370                 375                 380

Tyr Thr Pro Asp Thr Pro Ala Thr Arg Gln Val Met Ala Glu Val Asn
385                 390                 395                 400

Lys Thr Phe Gln Glu Leu Ala Val Phe His Asp Leu Glu Gly Met Trp
                405                 410                 415

Glu Glu Leu Ser Pro Lys Ile Trp Thr Phe Met Glu Asn Ser Gln Glu
            420                 425                 430

Met Asp Leu Val Arg Met Leu Leu Asp Ser Arg Asp Asn Asp His Phe
        435                 440                 445

Trp Glu Gln Gln Leu Asp Gly Leu Asp Trp Thr Ala Gln Asp Ile Val
    450                 455                 460

Ala Phe Leu Ala Lys His Pro Glu Asp Val Gln Ser Ser Asn Gly Ser
465                 470                 475                 480

Val Tyr Thr Trp Arg Glu Ala Phe Asn Glu Thr Asn Gln Ala Ile Arg
                485                 490                 495

Thr Ile Ser Arg Phe Met Glu Cys Val Asn Leu Asn Lys Leu Glu Pro
            500                 505                 510

Ile Ala Thr Glu Val Trp Leu Ile Asn Lys Ser Met Glu Leu Leu Asp
        515                 520                 525

Glu Arg Lys Phe Trp Ala Gly Ile Val Phe Thr Gly Ile Thr Pro Gly
    530                 535                 540

Ser Ile Glu Leu Pro His His Val Lys Tyr Lys Ile Arg Met Asp Ile
545                 550                 555                 560

Asp Asn Val Glu Arg Thr Asn Lys Ile Lys Asp Gly Tyr Trp Asp Pro
                565                 570                 575

Gly Pro Arg Ala Asp Pro Phe Glu Asp Met Arg Tyr Val Trp Gly Gly
            580                 585                 590

Phe Ala Tyr Leu Gln Asp Val Val Glu Gln Ala Ile Ile Arg Val Leu
        595                 600                 605

Thr Gly Thr Glu Lys Lys Thr Gly Val Tyr Met Gln Gln Met Pro Tyr
    610                 615                 620

Pro Cys Tyr Val Asp Asp Ile Phe Leu Arg Val Met Ser Arg Ser Met
625                 630                 635                 640

Pro Leu Phe Met Thr Leu Ala Trp Ile Tyr Ser Val Ala Val Ile Ile
                645                 650                 655

Lys Gly Ile Val Tyr Glu Lys Glu Ala Arg Leu Lys Glu Thr Met Arg
            660                 665                 670

Ile Met Gly Leu Asp Asn Ser Ile Leu Trp Phe Ser Trp Phe Ile Ser
        675                 680                 685

Ser Leu Ile Pro Leu Leu Val Ser Ala Gly Leu Leu Val Val Ile Leu
    690                 695                 700

Lys Leu Gly Asn Leu Leu Pro Tyr Ser Asp Pro Ser Val Val Phe Val
705                 710                 715                 720

Phe Leu Ser Val Phe Ala Val Val Thr Ile Leu Gln Cys Phe Leu Ile
                725                 730                 735

Ser Thr Leu Phe Ser Arg Ala Asn Leu Ala Ala Ala Cys Gly Gly Ile
            740                 745                 750

Ile Tyr Phe Thr Leu Tyr Leu Pro Tyr Val Leu Cys Val Ala Trp Gln
        755                 760                 765

Asp Tyr Val Gly Phe Thr Leu Lys Ile Phe Ala Ser Leu Leu Ser Pro
    770                 775                 780

Val Ala Phe Gly Phe Gly Cys Glu Tyr Phe Ala Leu Phe Glu Glu Gln
785                 790                 795                 800

Gly Ile Gly Val Gln Trp Asp Asn Leu Phe Glu Ser Pro Val Glu Glu
                805                 810                 815

Asp Gly Phe Asn Leu Thr Thr Ser Val Ser Met Met Leu Phe Asp Thr
            820                 825                 830

Phe Leu Tyr Gly Val Met Thr Trp Tyr Ile Glu Ala Val Phe Pro Gly
        835                 840                 845

Gln Tyr Gly Ile Pro Arg Pro Trp Tyr Phe Pro Cys Thr Lys Ser Tyr
    850                 855                 860

Trp Phe Gly Glu Glu Ser Asp Glu Lys Ser His Pro Gly Ser Asn Gln
865                 870                 875                 880

Lys Arg Ile Ser Glu Ile Cys Met Glu Glu Glu Pro Thr His Leu Lys
                885                 890                 895

Leu Gly Val Ser Ile Gln Asn Leu Val Lys Val Tyr Arg Asp Gly Met
            900                 905                 910

Lys Val Ala Val Asp Gly Leu Ala Leu Asn Phe Tyr Glu Gly Gln Ile
        915                 920                 925

Thr Ser Phe Leu Gly His Asn Gly Ala Gly Lys Thr Thr Thr Met Ser
    930                 935                 940

Ile Leu Thr Gly Leu Phe Pro Pro Thr Ser Gly Thr Ala Tyr Ile Leu
945                 950                 955                 960

Gly Lys Asp Ile Arg Ser Glu Met Ser Thr Ile Arg Gln Asn Leu Gly
                965                 970                 975

Val Cys Pro Gln His Asn Val Leu Phe Asp Met Leu Thr Val Glu Glu
            980                 985                 990

His Ile Trp Phe Tyr Ala Arg Leu Lys Gly Leu Ser Glu Lys His Val
        995                 1000 .              1005

Lys Ala Glu Met Glu Gln Met Ala Leu Asp Val Gly Leu Pro Ser Ser
    1010                1015                1020

Lys Leu Lys Ser Lys Thr Ser Gln Leu Ser Gly Gly Met Gln Arg Lys
1025                1030                1035                1040

Leu Ser Val Ala Leu Ala Phe Val Gly Gly Ser Lys Val Val Ile Leu
                1045                1050                1055

Asp Glu Pro Thr Ala Gly Val Asp Pro Tyr Ser Arg Arg Gly Ile Trp
            1060                1065                1070

Glu Leu Leu Leu Lys Tyr Arg Gln Gly Arg Thr Ile Ile Leu Ser Thr
        1075                1080                1085

His His Met Asp Glu Ala Asp Val Leu Gly Asp Arg Ile Ala Ile Ile
    1090                1095                1100

Ser His Gly Lys Leu Cys Cys Val Gly Ser Ser Leu Phe Leu Lys Asn
1105                1110                1115                1120

Gln Leu Gly Thr Gly Tyr Tyr Leu Thr Leu Val Lys Lys Asp Val Glu
                1125                1130                1135

Ser Ser Leu Ser Ser Cys Arg Asn Ser Ser Ser Thr Val Ser Tyr Leu
            1140                1145                1150

Lys Lys Glu Asp Ser Val Ser Gln Ser Ser Ser Asp Ala Gly Leu Gly
        1155                1160                1165

Ser Asp His Glu Ser Asp Thr Leu Thr Ile Asp Val Ser Ala Ile Ser
    1170                1175                1180

Asn Leu Ile Arg Lys His Val Ser Glu Ala Arg Leu Val Glu Asp Ile
1185                1190                1195                1200

Gly His Glu Leu Thr Tyr Val Leu Pro Tyr Glu Ala Ala Lys Glu Gly
                1205                1210                1215

Ala Phe Val Glu Leu Phe His Glu Ile Asp Asp Arg Leu Ser Asp Leu
            1220                1225                1230

Gly Ile Ser Ser Tyr Gly Ile Ser Glu Thr Thr Leu Glu Glu Ile Phe
        1235                1240                1245

Leu Lys Val Ala Glu Glu Ser Gly Val Asp Ala Glu Thr Ser Asp Gly
    1250                1255                1260

Thr Leu Pro Ala Arg Arg Asn Arg Arg Ala Phe Gly Asp Lys Gln Ser
1265                1270                1275                1280

Cys Leu Arg Pro Phe Thr Glu Asp Asp Ala Ala Asp Pro Asn Asp Ser
                1285                1290                1295

Asp Ile Asp Pro Glu Ser Arg Glu Thr Asp Leu Leu Ser Gly Met Asp
            1300                1305                1310

Gly Lys Gly Ser Tyr Gln Val Lys Gly Trp Lys Leu Thr Gln Gln Gln
        1315                1320                1325

Phe Val Ala Leu Leu Trp Lys Arg Leu Leu Ile Ala Arg Arg Ser Arg
    1330                1335        .       1340

Lys Gly Phe Phe Ala Gln Ile Val Leu Pro Ala Val Phe Val Cys Ile
1345                1350                1355                1360

Ala Leu Val Phe Ser Leu Ile Val Pro Pro Phe Gly Lys Tyr Pro Ser
                1365                1370                1375

Leu Glu Leu Gln Pro Trp Met Tyr Asn Glu Gln Tyr Thr Phe Val Ser
            1380                1385                1390

Asn Asp Ala Pro Glu Asp Thr Gly Thr Leu Glu Leu Leu Asn Ala Leu
        1395                1400                1405

Thr Lys Asp Pro Gly Phe Gly Thr Arg Cys Met Glu Gly Asn Pro Ile
    1410                1415                1420

Pro Asp Thr Pro Cys Gln Ala Gly Glu Glu Glu Trp Thr Thr Ala Pro
1425                1430                1435                1440

Val Pro Gln Thr Ile Met Asp Leu Phe Gln Asn Gly Asn Trp Thr Met
                1445                1450                1455

Gln Asn Pro Ser Pro Ala Cys Gln Cys Ser Ser Asp Lys Ile Lys Lys
            1460                1465                1470

Met Leu Pro Val Cys Pro Pro Gly Ala Gly Gly Leu Pro Pro Pro Gln
        1475                1480                1485

Arg Lys Gln Asn Thr Ala Asp Ile Leu Gln Asp Leu Thr Gly Arg Asn
    1490                1495                1500

Ile Ser Asp Tyr Leu Val Lys Thr Tyr Val Gln Ile Ile Ala Lys Ser
1505                1510                1515                1520

Leu Lys Asn Lys Ile Trp Val Asn Glu Phe Arg Tyr Gly Gly Phe Ser
                1525                1530                1535

Leu Gly Val Ser Asn Thr Gln Ala Leu Pro Pro Ser Gln Glu Val Asn
            1540                1545                1550

Asp Ala Thr Lys Gln Met Lys Lys His Leu Lys Leu Ala Lys Asp Ser
        1555                1560                1565

Ser Ala Asp Arg Phe Leu Asn Ser Leu Gly Arg Phe Met Thr Gly Leu
    1570                1575                1580

Asp Thr Arg Asn Asn Val Lys Val Trp Phe Asn Asn Lys Gly Trp His
1585                1590                1595                1600

Ala Ile Ser Ser Phe Leu Asn Val Ile Asn Asn Ala Ile Leu Arg Ala
                1605                1610                1615

Asn Leu Gln Lys Gly Glu Asn Pro Ser His Tyr Gly Ile Thr Ala Phe
            1620                1625                1630

Asn His Pro Leu Asn Leu Thr Lys Gln Gln Leu Ser Glu Val Ala Pro
        1635                1640                1645

Met Thr Thr Ser Val Asp Val Leu Val Ser Ile Cys Val Ile Phe Ala
    1650                1655                1660

Met Ser Phe Val Pro Ala Ser Phe Val Val Phe Leu Ile Gln Glu Arg
1665                1670                1675                1680

Val Ser Lys Ala Lys His Leu Gln Phe Ile Ser Gly Val Lys Pro Val
                1685                1690                1695

Ile Tyr Trp Leu Ser Asn Phe Val Trp Asp Met Cys Asn Tyr Val Val
            1700                1705                1710

Pro Ala Thr Leu Val Ile Ile Ile Phe Ile Cys Phe Gln Gln Lys Ser
        1715                1720                1725

Tyr Val Ser Ser Thr Asn Leu Pro Val Leu Ala Leu Leu Leu Leu Leu
    1730                1735                1740

Tyr Gly Trp Ser Ile Thr Pro Leu Met Tyr Pro Ala Ser Phe Val Phe
1745                1750                1755                1760

Lys Ile Pro Ser Thr Ala Tyr Val Val Leu Thr Ser Val Asn Leu Phe
                1765                1770                1775

Ile Gly Ile Asn Gly Ser Val Ala Thr Phe Val Leu Glu Leu Phe Thr
            1780                1785                1790

Asp Asn Lys Leu Asn Asn Ile Asn Asp Ile Leu Lys Ser Val Phe Leu
        1795                1800                1805

Ile Phe Pro His Phe Cys Leu Gly Arg Gly Leu Ile Asp Met Val Lys
    1810                1815                1820

Asn Gln Ala Met Ala Asp Ala Leu Glu Arg Phe Gly Glu Asn Arg Phe
1825                1830                1835                1840

Val Ser Pro Leu Ser Trp Asp Leu Val Gly Arg Asn Leu Phe Ala Met
                1845                1850                1855

Ala Val Glu Gly Val Val Phe Phe Leu Ile Thr Val Leu Ile Gln Tyr
            1860                1865                1870

Arg Phe Phe Ile Arg Pro Arg Pro Val Asn Ala Lys Leu Ser Pro Leu
        1875                1880                1885

Asn Asp Glu Asp Glu Asp Val Arg Arg Glu Arg Gln Arg Ile Leu Asp
    1890                1895                1900

Gly Gly Gly Gln Asn Asp Ile Leu Glu Ile Lys Glu Leu Thr Lys Ile
1905                1910                1915                1920

Tyr Arg Arg Lys Arg Lys Pro Ala Val Asp Arg Ile Cys Val Gly Ile
                1925                1930                1935

Pro Pro Gly Glu Cys Phe Gly Leu Leu Gly Val Asn Gly Ala Gly Lys
            1940                1945                1950

Ser Ser Thr Phe Lys Met Leu Thr Gly Asp Thr Thr Val Thr Arg Gly
        1955                1960                1965

Asp Ala Phe Leu Asn Arg Asn Ser Ile Leu Ser Asn Ile His Glu Val
    1970                1975                1980

His Gln Asn Met Gly Tyr Cys Pro Gln Phe Asp Ala Ile Thr Glu Leu
1985                1990                1995                2000

Leu Thr Gly Arg Glu His Val Glu Phe Phe Ala Leu Leu Arg Gly Val
                2005                2010                2015

Pro Glu Lys Glu Val Gly Lys Val Gly Glu Trp Ala Ile Arg Lys Leu
            2020                2025                2030

Gly Leu Val Lys Tyr Gly Glu Lys Tyr Ala Gly Asn Tyr Ser Gly Gly
        2035                2040                2045

Asn Lys Arg Lys Leu Ser Thr Ala Met Ala Leu Ile Gly Gly Pro Pro
    2050                2055                2060

Val Val Phe Leu Asp Glu Pro Thr Thr Gly Met Asp Pro Lys Ala Arg
2065                2070                2075                2080

Arg Phe Leu Trp Asn Cys Ala Leu Ser Val Val Lys Glu Gly Arg Ser
                2085                2090                2095

Val Val Leu Thr Ser His Ser Met Glu Glu Cys Glu Ala Leu Cys Thr
            2100                2105                2110

Arg Met Ala Ile Met Val Asn Gly Arg Phe Arg Cys Leu Gly Ser Val
        2115                2120                2125

Gln His Leu Lys Asn Arg Phe Gly Asp Gly Tyr Thr Ile Val Val Arg
    2130                2135                2140

Ile Ala Gly Ser Asn Pro Asp Leu Lys Pro Val Gln Asp Phe Phe Gly
2145                2150                2155                2160

Leu Ala Phe Pro Gly Ser Val Pro Lys Glu Lys His Arg Asn Met Leu
                2165                2170                2175

Gln Tyr Gln Leu Pro Ser Ser Leu Ser Ser Leu Ala Arg Ile Phe Ser
            2180                2185                2190

Ile Leu Ser Gln Ser Lys Lys Arg Leu His Ile Glu Asp Tyr Ser Val
        2195                2200                2205

Ser Gln Thr Thr Leu Asp Gln Val Phe Val Asn Phe Ala Lys Asp Gln
    2210                2215                2220

Ser Asp Asp Asp His Leu Lys Asp Leu Ser Leu His Lys Asn Gln Thr
2225                2230                2235                2240

Val Val Asp Val Ala Val Leu Thr Ser Phe Leu Gln Asp Glu Lys Val
                2245                2250                2255

Lys Glu Ser Tyr Val
            2260

## Description

### Background of the invention

Epidemiological studies have established that plasma high density lipoprotein cholesterol (HDL-C) concentrations are inversely associated with the incidence of coronary heart disease (CHD) (1-4). Moreover, HDL deficiency is the most common lipid abnormality observed in patients with premature CHD (5,6), with nearly half of all CHD patients having low concentrations of HDL-C. Genetic factors account for approximately 50% of the variation in HDL-C levels in the general population (7), with several common mutations identified in genes encoding for proteins involved in the regulation of plasma HDL-C concentrations and, ultimately, playing a role in the development of CHD (8).

Recently, we (9,10) and others (11-15) demonstrated that familial HDL deficiency (FDH) syndromes with either splenomegalie or atherosclerosis (e.g. Tangier disease (TD); familial hypoalphaglycoproteinaemia) are rare HDL-deficiencies caused by mutations in the gene encoding the ATP-binding cassette transporter-1 (ABC1). ABC1 is a member of the ATP binding cassette family of proteins that are involved in the transmembrane transport of a variety of different molecules, including bile acids, steroid hormones, ions, amino acids, sugars and vitamins (16-19). TD is characterized by severely diminished plasma HDL-C concentrations, reduced levels of low density lipoprotein cholesterol (LDL-C), the deposition of cholesteryl esters in the reticulo-endothelial system with splenomegaly and enlargement of tonsils and lymph nodes (20), as well as a predisposition to neuropathy and CHD (21,22). The HDL deficiency in these patients has been related to a rapid catabolism of HDL and a decrease in ApoA-I induced lipid efflux from various cells including mononuclear phagocytes (23-25). Familial hypoalphaglycoproteinaemia represents the other clinical phenotype that is characterized by a similar disturbance in ApoA-I metabolism but is associated with an enhanced risk of CHD rather than splenomegalie. ApoA-I mediated cellular cholesterol efflux is the first step in the reverse cholesterol transport and prevents excessive cholesterol accumulation in peripheral cells. In addition, this process is suggested to contribute to the formation of mature HDL particles, thereby influencing the pool size of HDL in the plasma.

Although mutations in *ABC1* cause TD, the precise molecular mechanism by which they do so remains unknown. Also, little is known about the association of ABC1 and HDL metabolism in individuals not affected with TD. Previous investigations on the function of ABC1 in mice have revealed its role in the engulfment of apoptotic cells by macrophages (26) and in macrophage interleukin-1β secretion (27). We have further shown that ABC1 is a cholesterol-responsive modulator of cellular lipid efflux (10) and, thus, plays a significant role in cholesterol homeostasis. Moreover, the ABC1-knockout mice (ABC1-/-) show massively reduced levels of serum lipids and lipoproteins. The expression of ABC1 in mucosa cells of the small intestine and the altered lipoprotein metabolism in ABC1-/- mice allows the conclusion that ABC1 plays also a major role in intestinal resorption and translocation of lipids into the lymph-system.

### Summary of the invention

Four different polymorphisms in *ABC1* (G596A, T1136C, A2589G, and G3456C) were identified in different Tangier kindreds as well as 516 healthy control subjects. Each of these polymorphisms affect the primary structure of the gene product.

A frequent coincidence of the G596A and A2589G polymorphisms was identified indicating that those two mutations are in linkage disequilibrium.

The two most common polymorphisms (G596A and A2589G) are both associated with a decreased in vitro ApoA-I mediated efflux of cholesterol from mononuclear phagocytes emphasizing the role of ABC1 in lipid efflux, the first step in reverse cholesterol transport. These results also are evidence that common polymorphisms in *ABC1* directly affect cellular lipid homeostasis, which is a key factor in the atherogenic process. A reduction of ApoA-I mediated efflux of cholesterol from mononuclear phagocytes is also a typical feature of Tangier disease.

Plasma lipoproteins, and HDL-cholesterol in particular, are not affected, probably because the effect of the *ABC1* polymorphisms on the protein function is compensated by other factors controlling plasma HDL levels.

Three common variants in the gene encoding ABC1 (G596A, A2589G, and G3456C) occur with significantly increased frequency in a population of men having low HDL-C as their primary lipid abnormality and established CHD, providing evidence for a role of *ABC1* in the premature CHD associated with common HDL deficiency states.

In summary, the identified polymorphisms in the ABC1 gene can be used for diagnosis and therapy of lipid disorders, cardiovascular diseases, and inflammatory diseases.

### Abbreviations

- ABC: ATP-binding cassette
- ANC: Anchor
- ApoA: Apolipoprotein A
- ApoB: Apolipoprotein B
- ApoE: Apolipoprotein E
- ATP: Adenosine triphosphate
- BMI: Body mass index
- bp: Base pairs
- cAMP: Cyclic adenosin monophosphat
- cDNA: Copy DNA
- CH: Cholesterol
- CHD: Coronary heart disease
- DET: Detection
- DPM: Disintegrations per minute
- E-LDL: Enzymatically modified LDL
- f: Forward
- Fluo: Fluorescein
- FOS: Framingham Offspring Study
- HDL: High density lipoprotein
- HDL-C: HDL-cholesterol
- kDa: Kilo Dalton
- LDL: Low density lipoprotein
- LDL-C: LDL-cholesterol
- M-CSF: Macrophage colony stimulating factor
- ph: Phosphorylation
- r: Reverse
- SD: Standard seviation
- SDS: Sodium dodecyl sulfate
- TD: Tangier Disease
- TG: Triglycerides
- VA-HIT: Veterans Affairs Cooperative HDL Cholesterol Intervention Trial
- wt: Wild type

### 1. References cited

1. Gordon, T., Castelli, W.P., Hjortland, M.C., Kannel, W.B., Dawber, T.R. High density lipoprotein as a protective factor against coronary heart disease. The Framingham Study. *Am. J. Med.* 1977; **62**: 707-14.
2. Kannel, W.B. High density lipoproteins: epidemiologic profile and risks of coronary artery disease. *Am. J. Cardiol.* 1983; **52**: 98-128.
3. Goldbourt, U., Yaari, S., Medalie, J.H. Isolated low HDL cholesterol as a risk factor for coronary heart disease mortality: a 21-year follow-up of 8000 men. *Arterioscler. Thromb. Vasc. Biol.* 1997; **17**: 107-13.
4. Wilson, P.W.F., D'Agostino, R.B., Levy, D., Belanger, A.M., Silbershatz, H., Kannel, W.B. Prediction of coronary heart disease using risk factor categories. *Circulation.* 1998; **97**: 1837-47.
5. Genest, J., Jr., et al. Familial lipoprotein disorders in patients with premature coronary artery disease. *Circulation* 1992; **85**: 2025-33.
6. Genest, J., Jr., Bard, J.-M., Fruchart, J.-C., Ordovas, J.M., Schaefer, E.J. Familial hypoalphalipoproteinemia in premature coronary artery disease. *Arterioscler. Thromb.* 1993; **13**: 1728-37.
7. Heller, D.A., DeFaire, D., Pedersen, N.L., Dahlen, G. , McClearn, G.E. Genetic and environmental influences on serum lipid levels in twins. *N*. *Engl. J Med.* 1993; **328**: 1150-56.
8. Calabresi, L., Franceschini, G. High density lipoproteins and coronary heart disease: insights from mutations leading to low high density lipoprotein. *Curr. Opin. Lipidol.* 1997; **8**: 219-24.
9. Bodzioch, M., et al. The gene encoding ATP-binding cassette transporter 1 is mutated in Tangier disease. *Nat. Genet.* 1999; **22**: 347-51.
10. Langmann T, Klucken J, Reil M, Liebisch G, Luciani MF, Chimini G, Kaminski WE, Schmitz G. Molecular cloning of the human ATP-binding cassette transporter 1 (hABC1): evidence for sterol-dependent regulation in macrophages. *Biochem Biophys Res Commun* 1999; **257**:29-33
11. Brooks-Wilson, A., et al. Mutations in *ABC1* in Tangier disease and familial high-density lipoprotein deficiency. *Nat. Genet.* 1999; **22**: 336-45.
12. Rust, S., et al. Tangier disease is caused by mutations in the gene encoding ATP-binding cassette transporter 1. *Nat Genet.* 1999; **22**: 352-55.
13. Remaley, A.T., et al. Human ATP-binding cassette transporter 1 (*ABC1*): Genomic organization and identification of the genetic defect in the original Tangier disease kindred. *Proc. Natl. Acad. Sci.* 1999; **96**: 12685-90.
14. Lawn, R.M., et al. The Tangier disease gene product ABC1 controls the cellular apolipoprotein-mediated lipid removal pathway. *J Clin. Invest.* 1999; **104:** R25-R31.
15. Brousseau, M.E., et al. Novel mutations in the gene encoding ATP-binding cassette 1 in four Tangier disease kindreds. *J Lipid Res.* (in press).
16. Higgins CF. ABC transporters: from microorganisms to man. *Annu Rev Cell Biol* 1992; **8**:67-113
17. Higgins CF. Flip-flop: the transmembrane translocation of lipids. *Cell* 1994; **79**:393-5
18. van Helvoort A, Smith AJ, Sprong H, Fritzsche I, Schinkel AH, Borst P, van Meer G. MDR1 P-glycoprotein is a lipid translocase of broad specificity, while MDR3 P-glycoprotein specifically translocates phosphatidylcholine. *Cell* 1996; **87**:507-17
19. Dekkers DW, Comfurius P, Schroit AJ, Bevers EM, Zwaal RF. Transbilayer movement of NBD-labeled phospholipids in red blood cell membranes: outward-directed transport by the multidrug resistance protein 1 (MRP1). *Biochemistry* 1998; **37**:14833-7
20. Assmann G, Schmitz G & Brewer HB Jr. Familial high density lipoprotein deficiency: Tangier disease. In: Scriver C.R, Beaudet, AL, Sly, WS, Valle, D (eds): The metabolic basis of inherited disease. 6^{th} ed., McGraw-Hill, New York Chapt 1989; **50:** 1267-1282.
21. Assmann, G., von Eckardstein, A., Brewer, H.B., Jr. Familial high density lipoprotein deficiency: Tangier disease. *In The Metabolic and Molecular Basis of Inherited Disease* (eds Scriver, C.R. et al.) 2053-2072 (McGraw-Hill, New York, 1995).
22. Serfaty-Lacrosniere, C., et al. Homozygous Tangier disease and cardiovascular disease. *Atherosclerosis* 1994; **107**: 85-98.
23. Schmitz G, Assmann G, Robenek H & Brennhausen B. Tangier disease: A disorder of intracellular membrane traffic. *Proc. Natl. Acad. Sci. USA* 1985; **82**: 6305-6309.
24. ^{Rogler} G, Trümbach B, Klima B, Lackner KJ & Schmitz G High density lipoprotein-mediated efflux of newly synthesized cholesterol is impaired in fibroblasts from Tangier patients while membrane description and efflux of lysosomal cholesterol are normal. *Arterioscler. Thromb. Vasc. Biol.* 1995; **15**: 683-690.
25. ^{Francis} GA, Knopp RH & Oram JF Defective removal of cellular cholesterol and phospholipids by apolipoprotein A-I in Tangier Disease. *J Clin. Invest.* 1995; **96**: 78-87.
26. Luciani, M.F., Chimini, G. The ATP binding cassette transporter ABC1 is required for engulfment of corpses generated by apoptotic cell death. *EMBO J.* 1996; **15**: 226-35.
27. Hamon, Y., et al. Interleukin-1β secretion is impaired by inhibitors of the ATP binding cassette transporter, ABC1. *Blood* 1997; **90**: 2911-15.
28. Sakr SW, Williams DL, Stoudt GW, Phillips MC & Rothblat GH. Induction of cellular cholesterol efflux to lipid-free apolipoprotein A-I by cAMP. Biochim. Biophys. Acta 1999; **19**:1438:85-98.
29. Aslanidis C & Schmitz G. High speed apolipoprotein E genotyping and apolipoprotein B3500 mutation detection using real-time fluorescence PCR and melting curves. *Clin. Chem.* 1999; **45**: 1094-1097.
30. Aslanidis C, Nauck M & Schmitz G. High-speed prothrombin G A 20210 and methylenetetrahydrofolate reductase C T 677 mutation detection using real-time fluorescence PCR and melting curves. *Biotechniques* 1999; **27**: 234-236.
31. Bhakdi S, Dorweiler B, Kirchmann R, Torzewski J, Weise E, Tranum-Jensen J, Walev I, Wieland E. On the pathogenesis of atherosclerosis: enzymatic transformation of human low density lipoprotein to an atherogenic moiety. *J*. *Exp. Med.* 1995; **182**: 1959-1971.
32. Feinleib, M., Kannel, W.B., Garrison, R.J., McNamara, P.M., Castelli, W.P. The Framingham Offspring Study. Design and preliminary data. *Prev. Med.* 1975; **4**: 518-25.
33. Rubins, H.B., et al. Gemfibrozil for the secondary prevention of coronary heart disease in men with low levels of high density lipoprotein cholesterol. *N. Engl. J*. *Med.* 1999; **341**: 410-18.
34. Wittwer, C.T., Ririe, K.M., Andrew, R.V., David, D.A., Gundry, R.A., Balis, U.J. The LightCycler: A microvolume multisample fluorimeter with rapid temperature control. *BioTechniques* 1997; **22**: 176-81.
35. Reiser, A., Geyer, M., van Miltenburg, R., Nauck, M., Tabiti, K. Mutation detection using multi-color detection on the LightCycler system. *Biochemica* 1999; **2**: 12-15.
36. Schaefer, E.J., et al. Factors associated with low and elevated plasma high density lipoprotein cholesterol and apolipoprotein A-I levels in the Framingham Offspring Study. *J. Lipid Res.* 1994; **35**: 871-82.
37. McNamara, J.R., Schaefer, E.J. Automated enzymatic standardized lipid analyses for plasma and lipoprotein fractions. *Clin. Chim. Acta.* 1987; **166**: 1-8.
38. Warnick, G.R., Benderson, J., Albers, J.J. Dextran sulfate-Mg2+ precipitation procedure for quantitation of high-density lipoprotein cholesterol. *Clin. Chem.* 1982; **28**: 1379-88.
39. Friedewald, W.T., Levy, R.I., Fredrickson, D.S. Estimation of the concentration of low-density lipoprotein cholesterol in plasma, without use of the preparative ultracentrifuge. *Clin. Chem.* 1972; **18**: 499-502.
40. Orso, E., Broccardo, C., Kaminski, W.E., Bottcher, A., Liebisch, G., Drobnik, W., Gotz, A., Chambenoit, O., Diederich, W., Langmann, T., Spruss, T., Luciani, M.F., Rothe, G., Lackner, K.J., Chimini, G. Schmitz, G. Transport of lipids from golgi to plasma membrane is defective in tangier disease patients and Abc1-deficient mice. *Nat. Genet.* 2000, **24**:192-196
41. Smythe, J.A., Symonds, G. Gene therapeutic agents: The use of ribozymes, antisense, and RNA decoys for HIV-1 infection. *Inflamm. Res.* 1995, **44**:11-15

### Description of Tables:

### Table 1:

**Summary of the characteristics of the study population consisting of 516 healthy blood donors.** Data are expressed as mean ± SD. n indicates the number of analyzed individuals.

### Table 2:

**Frequency of *ABC1* polymorphisms G596A, A2589G, G3456C, and T1136C in healthy control subjects.** Genotyping was performed by LightCycler (Roche Diagnostics) PCR analysis. Data provide the relative percentage of homozygous, heterozygous or wild type individuals at the different polymorphic sites. n indicates the number of analyzed individuals.

### Table 3:

**Distribution of G596A and A2589G polymorphisms in healthy control subjects.** Data represent absolute numbers of subjects with the respective allele combination. Data in parenthesis provide the relative percentage within each group.

### Table 4:

**Amplification primers and hybridization probes for the LightCycler polymorphism analysis.** Hybridization probes are designated as detection (DET) or anchor (ANC) probes. As indicated, they are labeled with LightCycler-Red 640 or 705 and fluorescein, respectively. Phosphorylation (ph) of the 3'-end prevents the elongation of the detection probe. All detection probes recognize wild-type sequences, with the exception of the probe DET1136/mut. Fluo - fluorescein.

### Table 5:

**Distribution of lipid and lipoprotein values in healthy individuals with different *ABC1* polymorphisms**. HDL-C and ApoA-I levels in homozygous, heterozygous, and wild type male and female individuals are presented. Data are expressed as mean ± SD. n indicates the number of analyzed individuals.

### Table 6:

**Cholesterol and phopholipid efflux from mononuclear phagocytes isolated form healthy individuals homozygous for the wild type nucleotide at all four polymporphic sites and from healthy individuals homozygous for the polymorphic allele at position 596 or 2589**. Lipid efflux is expressed as percent of basal unstimulated efflux observed in serum-free macrophage medium without ApoA-I. Data are the mean ± SD of the indicated number (n) of different subjects. Efflux analysis for each subject was performed at least in quadruplicate. P values were calculated by independent Student T-test for wt/wt versus G596A and A2589G, respectively.

### Table 7:

**Characteristics of FOS and VA-HIT subjects.** Results are listed as means±SD. *Significantly different from male FOS control subjects, P<0.01.

### Table 8:

**Genotype distribution of the *ABC1* polymorphisms in FOS versus VA-HIT subjects.** Values in parentheses are expressed as a percentage of the total number of subjects within each group.

### Table 9:

**Association of the G596A and A2589G *ABC1* polymorphisms in FOS and VA-HIT subjects.** Data represent absolute numbers of subjects out of a total of 1,008 for FOS and 1,014 for VA-HIT, whereas values in parentheses (%) provide the relative percentage of subjects within each group (FOS or VA-HIT). Significant differences in proportions between FOS and VA-HIT are presented in bold face type.

### Table 10:

**Association of the G596A and G3456C *ABC1* polymorphisms in FOS and VA-HIT subjects.** Data represent absolute numbers of subjects out of a total of 1,006 for FOS and 1,014 for VA-HIT, whereas values in parentheses (%) provide the relative percentage of subjects within each group (FOS or VA-HIT). Significant differences in proportions between FOS and VA-HIT are presented in bold face type.

### Table 11:

**Association of the A2589G and G3456C *ABC1* polymorphisms in FOS and VA-HIT subjects.** Data represent absolute numbers of subjects out of a total of 1,008 for FOS and 1,014 for VA-HIT, whereas values in parentheses (%) provide the relative percentage of subjects within each group (FOS or VA-HIT). Significant differences in proportions between FOS and VA-HIT are presented in bold face type.

**Table 1:**

| **Summary of the characteristics of the study population consisting of 516 healthy blood donors.** | | | | | | |
|---|---|---|---|---|---|---|
| | n | Age | Cholesterol mmol/l | Triglycerides mmol/l | HDL-C mmol/l | LDL-C mmol/l |
| Men | 274 | 27.3 ± 7.7 | 4.95 ± 1.13 | 1.23 ± 0.85 | 1.43 ± 0.35 | 2.97 ± 1.01 |
| Women | 242 | 30.5 ± 10.2 | 5.63 ± 1.10 | 1.19 ± 0.56 | 1.72 ±0.45 | 3.24 ± 1.02 |

**Table 2:**

| **Frequency of ABC1 Polymorphisms in healthy control subjects** | | | | |
|---|---|---|---|---|
| | **n** | **Homozygous** | **Heterozygous** | **Wild Type** |
| **G596A** | 511 | 8.0 % | 43.5 % | 48.5 % |
| **A2589G** | 514 | 2.3 % | 22.8 % | 74.9 % |
| **G3456C** | 516 | 0.0 % | 5.0 % | 95.0 % |
| **T1136C** | 515 | 0.0 % | 0.6 % | 99.4 % |

**Table 3:**

| **Distribution of G596A and A2589G Polymorphisms in healthy control subjects** | | | |
|---|---|---|---|
| | **G596A** | | |
| | 596A/A | 596G/A | 596G/G |
| **A2589G** | | | |
| 2589G/G | 4 (9.8%) | 6 (2.7%) | 2 (0.8%) |
| 2589A/G | 14 (34.1%) | 61 (27.6%) | 39 (15.7%) |
| 2589A/A | 23 (56.1%) | 154 (69.7%) | 207 (83.5%) |

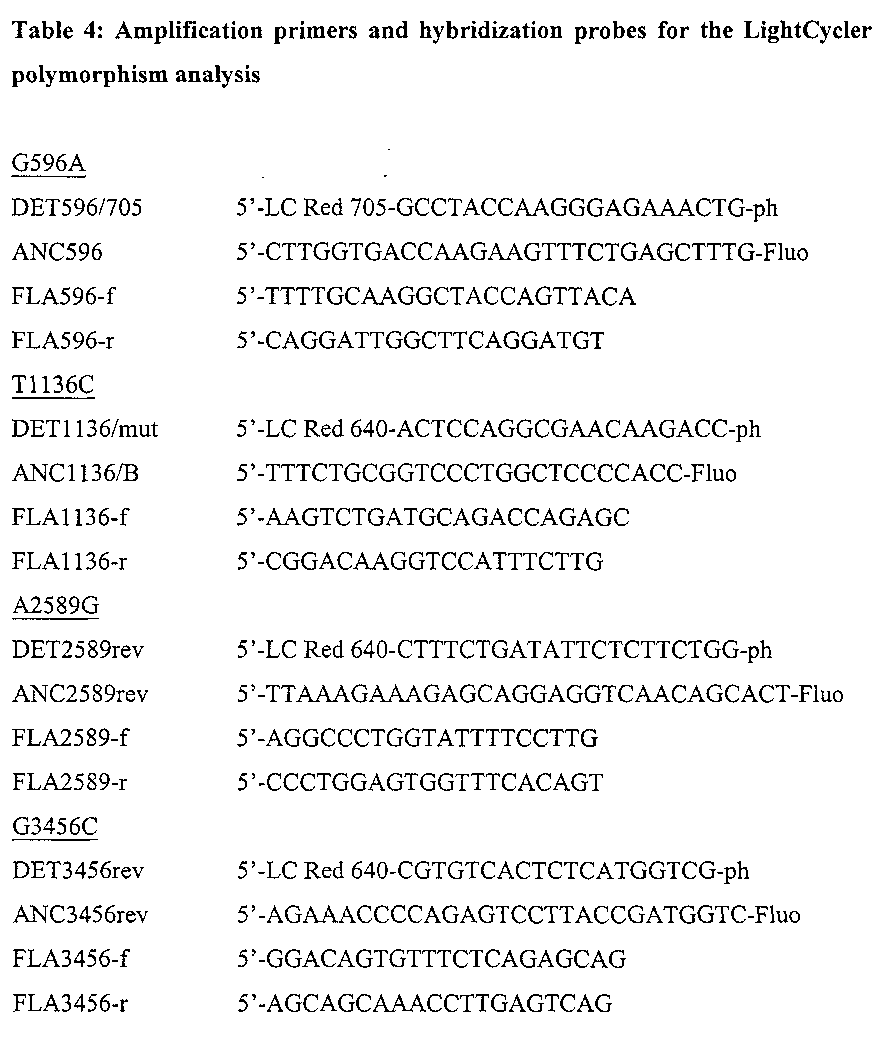

**Table 5:**

| **Distribution of lipid and lipoprotein values in healthy individuals with different *ABC1* polymorphisms** | | | |
|---|---|---|---|
| | Homozygous | Heterozygous | Wild Type |
| **G596A** | *596A/A* | *596G/A* | *596G/G* |
| ***men*** | n=19 | n=127 | n=126 |
| HDL-C (mmol/l) | 1.41 ± 0.26 | 1.45 ± 0.36 | 1.41 ± 0.34 |
| ApoA-I (g/l) | 1.51 ± 1.6 | 1.49 ± 2.5 | 1.48 ± 2.3 |
| ***women*** | n=22 | n=95 | n=122 |
| HDL-C (mmol/l) | 1.82 ± 0.45 | 1.75 ± 0.46 | 1.73 ± 0.44 |
| ApoA-I (g/l) | 1.77 ± 3.9 | 1.79 ± 3.6 | 1.76 ± 3.3 |

| **A2589G** | *2589G/G* | *2589A/G* | *2589A/A* |
|---|---|---|---|
| ***men*** | n=7 | n=54 | n=212 |
| HDL-C (mmol/l) | 1.50 ± 0.28 | 1.39 ± 0.32 | 1.44 ± 0.36 |
| ApoA-I (g/l) | 1.62 ± 21 | 1.47 ± 24 | 1.49 ± 24 |
| ***women*** | n=5 | n=63 | n=173 |
| HDL-C (mmol/l) | 1.94 ± 0.40 | 1.80 ± 0.37 | 1.72 ± 0.48 |
| ApoA-I (g/l) | 2.02 ± 28 | 1.79 ± 32 | 1.76 ± 36 |

| **G3456C** | *3456C/C* | *3456G/C* | *3456G/G* |
|---|---|---|---|
| ***men*** | n=0 | n=15 | n=228 |
| HDL-C (mmol/l) | | 1.37 ± 0.27 | 1.44 ± 0.35 |
| ApoA-I (g/l) | | 1.50 ± 26 | 1.49 ± 24 |
| ***women*** | n=0 | n=11 | n=262 |
| HDL-C (mmol/l) | | 1.84 ± 0.46 | 1.74 ± 0.45 |
| ApoA-I (g/l) | | 1.79 ± 34 | 1.77 ± 35 |

**Table 6:**

| **Cholesterol and phopholipid efflux from mononuclear phagocytes isolated form healthy individuals homozygous for the wild type nucleotide at all four polymporphic sites and from healthy individuals homozygous for the polymorphic allele at position 596 or 2589.** | | | |
|---|---|---|---|
| **A. Cholesterol efflux: ApoA-I [10 µg/ml]** | | | |
| | **wt/wt 596G/G; 2589A/A** | **596A/A** | **2589G/G** |
| *all probands* | 128.7 ± 17.5% | 97.7 ± 22.4% | 103.4 ± 18.7% |
| | (n=18) | (n=8; p<0.001) | (n=11; p<0.001) |
| *men* | 126.7 ± 16.6% | 98.8 ± 43.8% | 104.5 ± 23.0% |
| | (n=14) | (n=2; n.s.) | (n=6; p<0.025) |
| *women* | 135.6 ± 21.4% | 97.3 ± 18.0% | 102.0 ± 14.3% |
| | (n=4) | (n=6; p<0.001) | (n=5; p<0.025) |

| **B. Cholesterol efflux: ApoA-I [10 µg/ml]+ 24 h pretreatment with forskolin** | | | |
|---|---|---|---|
| **[10 µM]** | **wt/wt 596G/G; 2589A/A** | **596A/A** | **2589G/G** |
| *men and women* | 121.8 ± 16.4% | 126.7 ± 37.4% | 121,5 ± 38.8% |
| | (n=12) | (n=8; n.s.) | (n=10; n.s.) |

| **C. Phospholipid efflux: ApoA-I [10 µg/ml]** | | | |
|---|---|---|---|
| | **wt/wt 596G/G; 2589A/A** | **596A/A** | **2589G/G** |
| *men and women* | 125.9 ± 15.3% | 117.3 ± 27.1% | 114,9 ± 32.6% |
| | (n=18) | (n=8; n.s.) | (n=10; n.s.) |

| **D. Phospholipid efflux: ApoA-I [10 µg/ml]+ 24 h pretreatment with forskolin [10 µM]** | | | |
|---|---|---|---|
| | **wt/wt 596G/G; 2589A/A** | **596A/A** | **2589G/G** |
| *men and women* | 135.4 ± 20.2% | 136.7 ± 12.8% | 124.5 ± 25.8% |
| | (n=12) | (n=8; n.s.) | (n=10; n.s.) |

**Table 7:**

| **Characteristics of FOS and VA-HIT subjects** | | |
|---|---|---|
| | **FOS** | **VA-HIT** |
| ***N*** | 1014 | 1014 |
| **Age (years)** | 53±9 | 64±7* |
| **BMI (kg/m**^{**2**}**)** | 27.7±3.9 | 29±5* |
| **TC (mg/dL)** | 206±36 | 175±25* |
| **LDL-C (mg/dL)** | 136±33 | 111±23* |
| **HDL-C (mg/dL)** | 44±12 | 32±5* |
| **TG (mg/dL)** | 138±101 | 161±68 |
| **TC:HDL-C** | 5.1±1.6 | 5.7±1.1* |

| | | |
|---|---|---|
| *Significantly different from male FOS control subjects, P<0.01. | | |

**Table 8:**

| **Genotype distribution of the *ABC1* polymorphisms in FOS versus VA-HIT subjects** | | | |
|---|---|---|---|
| **Mutation** | **FOS** | **VA-HIT** | *P* |
| **G596A** | | | |
| Total *n* | 1013 | 1014 | |
| GG (%)* | 543 (53.6) | 454 (44.8) | |
| GA (%) | 402 (39.7) | 484 (47.7) | |
| AA (%) | 68 (6.7) | 76 (7.5) | |
| **A Allele frequency** | **0.266** | **0.314** | **0.000** |

| **A2589G** | | | |
|---|---|---|---|
| Total n | 1014 | 1014 | |
| AA (%) | 779 (76.8) | 707 (69.7) | |
| AG (%) | 222 (21.9) | 285 (28.1) | |
| GG (%) | 13 (1.3) | 22 (2.2) | |
| G Allele frequency | **0.122** | **0.162** | **0.001** |
| | | | |

| G3456C | | | |
|---|---|---|---|
| **Total *n*** | 1013 | 1014 | |
| GG (%) | 965 (95.3) | 940 (92.7) | |
| GC (%) | 43 (4.2) | 70 (6.9) | |
| CC (%) | 5 (0.5) | 4 (0.4) | |
| C Allele frequency | **0.026** | **0.038** | **0.032** |

**Table 9:**

| **Association of the G596A and A2589G *ABC1* polymorphisms in FOS and VA-HIT subjects** | | | | | | |
|---|---|---|---|---|---|---|
| **G596A** | | | | | | |
| | ***GG*** | | **GA** | | **AA** | |
| | FOS | **VA-HIT** | FOS | VA-HIT | FOS | VA-HIT |
| A2589G | | | | | | |
| **AA** | 459 | 360 | 77 | 89 | 5 | 5 |
| | **(45.6)** | **(35.5)** | (7.6) | (8.8) | (0.5) | (0.5) |
| | | | | | | |
| **AG** | 268 | 30 | 125 | 166 | 5 | 12 |
| | **(26.6)** | **(30.2)** | **(12.4)** | **(16.4)** | (0.5) | (1.2) |
| | | | | | | |
| **GG** | 47 | 41 | 18 | 30 | 3 | 5 |
| | (4.7) | (4.0) | **(1.8)** | **(3.0)** | (0.3) | (0.5) |

**Table 10:**

| **Association of the G596A and G3456C *ABC1* polymorphisms in FOS and VA-HIT subjects** | | | | | | |
|---|---|---|---|---|---|---|
| **G596A** | | | | | | |
| | ***GG*** | | **GA** | | **AA** | |
| | FOS | VA-HIT | FOS | VA-HIT | FOS | VA-HIT |
| **G3456C** | | | | | | |
| **GG** | 508 | 426 | 27 | 26 | 3 | 2 |
| | **(50.5)** | **(42.0)** | (2.7) | (2.6) | (0.3) | (0.2) |
| **GC** | 384 | 450 | 14 | 34 | 2 | 0 |
| | **(38.2)** | **(44.4)** | **(1.4)** | **(3.4)** | (0.2) | (0) |
| **CC** | 66 | 64 | 2 | 10 | 0 | 2 |
| | (6.6) | (6.3) | (0.2) | (1.0) | (0) | (0.2) |

**Table 11:**

| **Association of the A2589G and G3456C *ABC1* polymorphisms in FOS and VA-HIT subjects** | | | | | | |
|---|---|---|---|---|---|---|
| **A2589G** | | | | | | |
| | ***AA*** | | **AG** | | **GG** | |
| **G3456C** | | | | | | |
| **GG** | 746 | 666 | 204 | 257 | 11 | 17 |
| | **(74.0)** | **(65.7)** | **(20.2)** | **(25.3)** | **(1.1)** | **(1.7)** |
| **GC** | 26 | 39 | 15 | 27 | 1 | 4 |
| | (2.6) | (3.8) | **(1.5)** | **(2.7)** | (0.1) | (0.4) |
| **CC** | 3 | 2 | 2 | 1 | 0 | 1 |
| | (0.3) | (0.2) | (0.2) | (0.1) | (0) | (0.1) |

### Description of specific embodiments

### Identification of four polymorphisms in the gene encoding the human ATP-binding cassette transporter 1 (ABC1) and association of these polymorphisms with decreased ApoA-I mediated efflux of cholesterol

This invention provides the four polymorphisms G596A, T1136C, A2589G, and G3456C in the *ABC1* gene (*ABC1* gene see Figures 1 and 2), which affect the primary structure of the gene product. The frequencies of these *ABC1* polymorphisms were determined in a young and healthy population (Example 1).

The two most common polymorphisms (G596A and A2589G) are both associated with a decreased in vitro ApoA-I mediated efflux of cholesterol from mononuclear phagocytes, which is a typical feature of Tangier disease (Example 2).

No association of any of the polymorphisms were found with HDL-cholesterol, ApoA-I or any other lipoprotein fraction probably because the effect of the *ABC1* polymorphisms on the protein function is compensated by other factors controlling plasma HDL levels (Example 2 and 4)

The precise mechanism by which ABC1 promotes cellular lipid efflux is not completely defined. However, recently we could show that ABC1 is expressed on the plasma membrane and the Golgi complex, mediates ApoA-I associated export of cholesterol and phospholipids from the cell, and is regulated by cholesterol flux. Also, our results indicate that ABC1 is involved in lipid export processes involving vesicular budding between the Golgi and the plasma membrane. In addition to a possible function as direct lipid transporter, ABC1 may also be involved in the initiation of signal transduction processes stimulating intracellular lipid transport pathways (40). The finding that pretreatment of monocytes with forskolin, an cAMP elevating agent known to stimulate ApoA-I dependent lipid efflux (28), was able to normalize cholesterol and phospholipid efflux in 596A/A and 2589G/G subjects, may indicate that these gene variants affect the ABC1 function, which is related both to signaling and lipid transport. Since such a defect could be overcome by regulatory signals, this hypothesis may also provide a possible explanation for the lack of an effect on serum HDL levels.

In summary, these data emphasizes the role of ABC1 in lipid efflux, the first step in reverse cholesterol transport, and can be taken as evidence that common polymorphisms in *ABC1* directly affect cellular lipid homeostasis, which is a key factor in the atherogenetic process.

### Frequencies of the identified ABC1 gene polymorphisms in men with low HDL cholesterol levels and coronary heart disease

The frequencies of three of the common single nucleotide polymorphisms in *ABC1* (G596A, A2589G, and G3456C) were found to be significantly increased in the population comprised of men having HDL deficiency and established CHD (participants enrolled in the Veterans Affairs Cooperative HDL Cholesterol Intervention Trial ,VA-HIT) relative to control subjects from the Framingham Offspring Study (FOS). Moreover, associations were observed between *ABC1* polymorphisms and status, indicating that wild type alleles were consistently more prevalent in FOS than in VA-HIT but that mutant alleles were consistently more prevalent in VA-HIT (Example 3).

In summary, these data demonstrate that three of the common variants in the gene encoding ABC1 occur with significantly increased frequency in a population of men having low HDL-C as their primary lipid abnormality and established CHD. These results indicate that common mutations in the *ABC1* play a role in the development of CHD in the general population. These data also provide evidence for a role of *ABC1* in the premature CHD associated with common HDL deficiency states. All three *ABC1* variants are either conserved between human and mouse or are very similar as in the case of A2589G (isoleucine and valine, respectively), suggesting that each has an important functional role.

Furthermore, the identification of ABC1 as a transporter for IL-1β identifies this gene as a candidate gene for treatment of inflammatory diseases including rheumatoid arthritis and septic shock (27). The cytokine IL-1β is a broadly acting proinflammatory mediator that has been implicated in the pathogenesis of these diseases.

Moreover, we could demonstrate, that glyburide as an inhibitor of IL-1β secretion inhibits not only Caspase I mediated processing of pro-IL-1β and release of mature IL-1β but simultaneously inhibits ceramide formation from sphingomyelin mediated by neutral sphingomyelinase and thereby releases human fibroblasts from G₂-phase cell cycle arrest. These data provide a further mechanism indicative for a function of ABC1 in signalling and cellular lipid metabolism.

Autoimmune disorders that are associated with the antiphospholipid syndrome (e.g. lupus erythematodes) can be related to dysregulation of B-cell and T-cell function, aberrant antigen processing, or aberrations in the asymmetric distribution of membrane phospholipids. ABC-transporters are, besides their transport function, candidate genes for phospholipid translocases, floppases and scramblases that regulate phospholipid asymmetry (outer leaflet: PC+SPM; inner leaflet: PS+PE) of biological membranes [11]. There is considerable evidence for a dysregulation of members of the ABC-transporter family in patient cells. We conclude that these ABC-cassettes are also candidate genes for a genetic basis of antiphospholipid syndromes such as in Lupus erythematodes.

In summary, our findings emphasize how insight into the pathophysiology of a major disease process may be gleaned from a very rare genetic disorder.

Lipid disorders represent diseases characterized by changes in the lipid- and lipoproteinconcentrations in the organism. Cardiovascular diseases represent diseases of the heart and vessel system including coronary heart disease and atherosclerosis. Inflammatory diseases include diseases such as psoriasis and lupus erythematodes.

This invention is directed to the nucleotide sequences of the identified *ABC1* polymorphism variants, the corresponding protein sequences, antibodies, pharmaceutical compositions, diagnostic tests, vectors, and vector host systems according to the claims.

Our findings identify *ABC1* as a target for the diagnosis of lipid disorders, cardiovascular diseases , and inflammatory diseases.

Our findings further identify *ABC1* as a target for therapeutic strategies aimed at the prevention of lipid disorders, cardiovascular diseases, and inflammatory diseases.

The identified *ABC1* polymorphisms may create immunogenic protein sites that can be used for the generation of antibodies. These antibodies can be used for the detection of mutated ABC1-protein and can be used for the diagnosis of lipid disorders, cardiovascular diseases, and inflammatory diseases.

Therapeutic strategies also include the gene therapy of lipid disorders, cardiovascular diseases, and inflammatory diseases. For this gene therapy, a polynucleotid consisting of a transcription unit is used. The transcription unit consists of at least 9 consecutive nucleotides that are identical or substantial similiar to the ABC1 cDNA sequence. Alternatively, the transcription unit consists of at least 9 consecutive nucleotides leading to the production of antisense RNA of wild type ABC1 or ABC1 containing one or more of the identified polymorphisms. The transcription unit is combined with heterologous transcription regulating sequences, which regulate the transcription in human cells. The heterologous transcription regulating sequences include a promoter that is constitutively active in human cells. Alternatively, the heterologous transcription regulating sequences can contain a promoter that can be induced or repressed in human cells by the addition of a regulatory substance.

Another therapeutic approach would be the application of antisense molecules or ribozymes or RNA decoys directed towards the *ABC1* transcripts resulting in a modulation of the ABC1 expression. The methodology has been reviewed in (41).

This invention also includes a vector containing the above described transcription unit and transcription regulating sequences. Preferred vector is a virus, e.g. retrovirus, adenovirus, adeno-associated virus, herpes simplex virus, lentivirus, vaccinia virus, sindbis virus, semliki forest virus.

Preferred vectors are also plasmids.

The invention also includes a pharmaceutical preparation that contains the recombinant vectors, e.g. in a colloidal dispersion system. Preferred colloidal dispersion systems are liposomes or polylysin ligands.

The invention also includes pharmaceutical compositions that contain the above described recombinant vector constructs in combination with nontoxic, inert, pharmaceutically-suited carrier substances. Application of these pharmaceutical compositions can be locally or systemically (e.g. intravenous, intraarterious, intramuscular, subcutane, intradermal, anal, vaginal, nasal, transdermal, intraperitoneal, oral, or as aerosol).

The invention further includes recombinant host cells, especially recombinant eucaryotic host cells, that contain the above described recombinant vector constructs.

### Examples

### Example 1

### ATP-binding cassette transporter 1 (ABC1) gene polymorphisms

### Complete cDNA sequence of human ABC1

We have cloned the complete cDNA sequence of human *ABC1* (10). The nucleotide sequence of the complete coding region of the human ABC1 gene is shown in Fig. 1. The open reading frame of 6603 bp encodes a 2201 amino acid protein with a predicted molecular weight of 220 kDa. The protein sequence of the human ABC1 gene is shown in Fig. 2.

### Identification of four polymorphisms in human ABC1

Complete sequence analysis (by dye primer sequencing ?) of human *ABC1* in different (how many ?)Tangier kindred's revealed several polymorphisms, which were also found in healthy control subjects. These are G596A, T1136C, A2589G, and G3456C. All of them change the amino acid sequence of ABC1 and therefore may affect the function of this transporter.

### Frequency of polymorphisms in the general population

In order to determine the frequency of these four polymorphisms in the general population, we have tested 516 healthy blood donors. Table 1 summarizes the characteristics of the blood donors. The allele frequencies shown in table 2 demonstrate that G596A, A2589G and G3456C variants are quite common in a young, healthy population. The G596A polymorphism had the highest prevalence with an estimated frequency of 29.8% for the minor allele (596A), followed by A2589G with 13.7% (2589G), and G3456C with 2.5% (3456C). There was no significant deviation from the Hardy-Weinberg equilibrium. Only only 3 heterozygotes were found for T1136C in our group.

When we further analyzed the distribution of the polymorphisms in healthy blood donors, we detected a frequent coincidence of the G596A and A2589G polymorphisms (table 3). In homozygote individuals for the minor G596A allele (596A/A; n=41) the frequency of the alleles A and G of the A2589G polymorphism was 73.2% and 26.8% respectively, while in homozygotes for the major G596A allele (596G/G; n=248) the frequencies were 91.3% and 8.7% (p < 0.001). This indicates that the two mutations are in linkage disequilibrium in our population. While there was apparently no linkage disequilibrium between G596A and G3456C, the numbers were too small to exclude this with certainty (data not shown).

### Methods

*Sample collection.* Study subjects were recruited from healthy blood donors at the Institute for Clinical Chemistry (department of transfusion medicine) at the University of Regensburg. Samples from 516 individuals who gave consent to genetic analysis at the time of recruitment were available for the study. At the day of sample collection lipid parameters were determined and DNA-extraction was performed. A local review committee approved the Sample collection for genetic analysis.

*DNA extraction.* All DNA samples were isolated from whole blood with a QIAamp DNA Blood Maxi Kit (Qiagen) according to the manufacturer's recommendations.

*Detection of polymorphisms*. Genotype analysis was performed on the LightCycler (Roche Diagnostics) as previously described, with minor modifications (29,30). The amplification primers and hybridization probes are listed in table 4. The latter are designated as detection or anchor probes. They are labeled with LightCycler-Red 640 or 705 and fluorescein, respectively. We performed 40 amplification cycles under the following conditions: denaturation at 95°C for 120 s in the first step and 0 s in all subsequent steps, annealing at 57°C for 10 s, extension at 72°C for 15 s. This was followed by a temperature gradient melting curve analysis as described elsewhere (29,30). All detection probes recognize wild-type sequences, with the exception of the probe DET1136/mut, which is complementary to the sequence of the minor allele, i.e. 1136C. In order to assure specificity and accuracy of the LightCycler analysis 100 randomly selected samples were subjected to automated sequencing (ABI Prism 310 Genetic Analyzer), which proved full concordance of the results.

*Statistical Analysis*. To compare allele frequencies we employed chi-square test.

### Example 2

### ABC1 gene polymorphisms are associated with impaired ApoA-I mediated cholesterol efflux but not with decreased serum HDL or ApoA-1

In Tangier patients, mutations in *ABC1* are associated with severely decreased HDL-cholesterol and a lack or marked reduction of ApoA-I induced cholesterol and phospholipid efflux. Therefore, we hypothesized that HDL-cholesterol and ApoA-I levels as well as cholesterol and phospholipid efflux might differ between homozygous wild-type genotypes and carriers of one of the polymorphisms. Since the T1136C polymorphism was only found in heterozygous form in three individuals we did not include T1136C in our statistical analysis.

### Analysis of the association of the identified ABC1 gene polymorphisms with HDL-cholesterol, ApoA-I, or other lipoprotein fractions

We first analyzed the distribution of lipid and lipoprotein values in individuals with different genotypes. As indicated in table 5 neither heterozygous nor homozygous carriers of the G596A, A2589G, or G3456C polymorphisms had significantly different HDL-cholesterol or ApoA-I levels compared to the homozygous wild type probands. We also analyzed further parameters of plasmatic lipoprotein metabolism, even though this was not an a priori hypothesis. Total plasma cholesterol, triglycerides, and LDL-cholesterol were also not significantly different among the various genotypes.

### Analysis of the association of the identified ABC1 gene polymorphisms with cholesterol and phospholipid efflux

We then analyzed cholesterol and phospholipid efflux from mononuclear phagocytes isolated from individuals homozygous for the wild type nucleotide at all four polymorphic sites and from individuals homozygous for the polymorphic allele at position 596 or 2589. Monocytes from subjects homozygous for at least one polymorphic allele were found to have a highly significant reduction in ApoA-I mediated cholesterol efflux (table 6 A). Whereas in cholesterol loaded monocytes from wild type individuals ApoA-I incubation for 17 hrs increases cholesterol efflux to approximately 130% of baseline values, no stimulation of efflux was observed in subjects homozygous for G596A (596A/A) or A2589G (2589G/G). This finding was independent of gender. In order to investigate, whether the lack of cholesterol efflux could be overcome by regulatory mechanisms, cells were pre-incubated with forskolin, a cAMP-elevating agent, which is known to stimulate ApoA-I mediated lipid efflux (28). As shown in table 6 B forskolin treatment normalized cholesterol efflux in subjects with polymorphic alleles.

A tendency towards reduced ApoA-I mediated efflux of choline containing phospholipids was also correlated with the presence of both variant alleles but did not reach statistical significance (table 6 C). Similar to the ApoA-I mediated cholesterol efflux, forskolin pretreatment abolished the differences between wild type and 596A/A or 2589G/G, respectively (table 6 D).

### Methods

*Lipids and Lipoproteins.* Venous blood was taken from all blood donors after a 12-h fast. The samples were obtained in polypropylene tubes, centrifuged at 4000 x g for 10 min. Cholesterol, triglycerides, and HDL-cholesterol was measured by standard clinical chemistry protocols as previously described. LDL cholesterol was calculated by the Friedewald equation. ApoA-I was determined on a BNA nephelometer (Dade-Behring) with reagents from Dade-Behring.

*Cellular Lipid Efflux.* Human peripheral blood cells were isolated from lithium-heparin treated blood samples of adult, healthy volunteers. The peripheral blood mononuclear cell fraction was separated by density gradient centrifugation over Ficoll-Histopaque 1.007 (Sigma). Cells positive for CD2, CD7, CD16, CD19 and CD56 were removed by appropriate magnetic beads (Dynal) leaving > 80% pure monocytes. Isolated monocytes were then cultured at a density of 1.5x10⁵ cells/mL in polystyrene culture dishes (100x15mm Petri dish, Falcon) in a serum-free macrophage medium (Macrophage-SFM, Gibco Life Technologies) supplemented with 50ng/mL human recombinant M-CSF (R&D Systems). After an overnight incubation cells were cholesterol loaded for 24 hrs by addition of enzymatically modified LDL (E-LDL; 40 µg/ml), which was prepared as previously described (31). The incubation medium was further supplemented with ¹⁴C-cholesterol (1.5 µCi/ml) and ³H-choline (10µCi/ml) in order to label cellular cholesterol and choline containing phospholipids. In a subset of experiments cells were stimulated with 10 µM forskolin during the labeling period. After 24 hrs monocytes were washed and incubated for 17 hrs with or without 10 µg/ml ApoA-I (Sigma) in macrophage medium containing 0.1 % bovine serum albumin and 50ng/mL M-CSF. After incubation the media were removed and centrifuged at 800 x g to precipitate any detached cells. Cells were lysed in 0.2% SDS. Lipids were extracted by liquid extraction according to the method of Bligh and Dyer (13). ³H and ¹⁴C radioactivities of the total lipid extracts were measured by liquid scintillation counting in order to distinguish between ³H labeled phospholipids and ¹⁴C-cholesterol. Lipid efflux was calculated as percentage of disintegrations per minute (dpm) in medium divided by the sum of dpm recovered from medium and cells. The ApoA-I specific efflux is expressed a percent of basal unstimulated efflux observed in serum-free macrophage medium without ApoA-I.

*Statistical Analysis.* For comparison of lipid parameters and efflux data between wild type and polymorphisms the two-sample t test was used.

### Example 3

### Frequencies of the identified ABC1 gene polymorphisms in men with low HDL cholesterol levels and coronary heart disease

To adress the question whether the identified polymorphisms in *ABC-1* can provide insight into common HDL deficiency states and CHD risk, we compared the frequencies of three common *ABC1* variants (G596A, A2589G, and G3456C) between two different populations.

One population consisted of men participating in the Framingham Offspring Study (FOS), a long-term prospective evaluation of cardiovascular disease risk factors (32), whereas the other population consisted of men enrolled in the Veterans Affairs Cooperative HDL Cholesterol Intervention Trial (VA-HIT). VA-HIT, a multicenter study initiated in 1991, was designed to address the hypothesis that increasing HDL-C levels with gemfibrozil therapy would reduce the incidence of death from CHD and nonfatal myocardial infarction in men with established CHD, who had reduced HDL-C concentrations (≤40mg/dL; 1 mmol/L) and normal LDL-C levels (33). The characteristics of these two groups are provided in table 7. Men participating in VA-HIT were older, and slightly heavier, than men in FOS. With respect to plasma lipids, men in VA-HIT had significantly lower concentrations of plasma total cholesterol (-15%), HDL-C (-27%), and LDL-C (-18%), with the greatest difference between the groups noted in HDL-C levels. These differences resulted in a TC:HDL-C ratio which was significantly increased in VA-HIT relative to FOS.in control subjects from the FOS with those of participants in the VA-HIT.

### Screening of the FOS and VA-HIT populations for the presence of the identified ABC1 polymorphisms

In order to test the hypothesis that *ABC1* polymorphisms may play a role in common HDL deficiency states and, ultimately, CHD risk, genomic DNA from 1,014 men per FOS and VA-HIT population, respectively, was screened for the presence of three common single nucleotide polymorphisms in the *ABC1* gene (G596A, A2589G, and G3456C), using sequence-specific primers with the LightCycler System. The frequency distribution of each *ABC1* polymorphism in the FOS and VA-HIT populations is provided in table 8. With regard to the G596A polymorphism, the frequency of the mutant A allele was significantly increased (P=0.000) in VA-HIT subjects (0.314) relative to FOS (0.266), translating into fewer wild type, but more heterozygous, individuals in the former group. This was similarly the case for the A2589G mutation in which the mutant G allele frequency was 0.162 in VA-HIT versus 0.122 in FOS (P<0.001). The G3456C polymorphism was rare in both the FOS and VA-HIT populations; however, a significant increase (P<0.032) in the frequency of the mutant C allele was observed in VA-HIT subjects (0.038 vs. 0.026). Thus, the frequency of each of these single nucleotide polymorphisms was significantly increased in a population comprised of men having HDL deficiency and established CHD.

### Analysis of the associations between each ABC1 polymorphism and status

We further evaluated these data for the presence of associations between each *ABC1* polymorphism and status. Associations between the G596A and A2589G, G596A and G3456C, and A2589G and G3456C *ABC1* variants in FOS and VA-HIT subjects are presented in tables 9-11, respectively. In each case, a significantly greater proportion of FOS subjects possessed a pair of wild type alleles relative to VA-HIT subjects. In contrast, a significantly greater proportion of VA-HIT subjects possessed either one or two mutant alleles for each *ABC1* variant. These data indicate that wild type alleles were consistently more prevalent in FOS than in VA-HIT, with mutant alleles consistently more prevalent in VA-HIT relative to FOS, suggesting that the occurrence of these *ABC1* variants may be linked.

### Methods

*Subjects.* Subjects were men participating in either the Framingham Offspring (n=1,014) or VA-HIT (n=1,014) studies. The design and methods for FOS have been described elsewhere in detail (32), as have the rationale, design, and results of the VA-HIT study (33). FOS subjects were participants in cycle 4 of FOS and were free of clinical evidence of CHD. For VA-HIT, men were recruited at 20 Veterans Affairs medical centers throughout the United States. Eligibility for the trial required a documented history of CHD, an age of <74 years, an absence of coexisting conditions, an HDL-C level of ≤40 mg/dL (1.0 mmol/L), an LDL-C of ≤140 mg/dL (3.6 mmol/L), and a triglyceride level of ≤300 mg/dL (3.4 mmol/L). Since DNA was available from 1,014 men participating in the VA-HIT study, an equal number of men participating in cycle 4 of FOS was randomly selected from a total of 1,139 samples available for analysis. Informed consent was obtained from all subjects. Nearly all of the subjects in both groups were Caucasians. Information on alcohol consumption, smoking, blood pressure, body mass index (BMI), and diabetes were available for all subjects enrolled in these studies. Plasma lipid and lipoprotein data used in our analyses were obtained from FOS subjects who were not taking a lipid-lowering medication, while those from the VA-HIT subjects were obtained at baseline, prior to randomization to a treatment (gemfibrozil, 600 mg bid) or placebo group.

*DNA Isolation.* Genomic DNA was extracted from whole blood samples using either QIAamp mini kits (Qiagen) or Generation Capture Column® kits (Gentra Systems).

***Mutation detection****.* Genotype analysis was performed using LightCycler technology (Roche Diagnostics), as described elsewhere in detail (28,34,35). Briefly, this method enables recognition of alterations in a nucleotide sequence through the detection of changes in fluorescence emission, dependent upon energy transfer between fluorophores on two adjacent hybridization probes. Both probes recognize the sequence of a specific amplicon. The detection probe, labeled on its 5' end with LightCycler-Red 640 or 705, overlaps the site of a polymorphic nucleotide, whereas the anchor probe, carrying fluorescein at the 3' end, binds to the amplicon 1 to 3 nucleotides upstream of the detection probe. After PCR in the presence of target DNA, samples are briefly denatured at 95°C, rapidly cooled to 35-40°C, and, in the final step, heated gradually to 70-80°C. Differentiation between a wild type and polymorphic allele is possible, because the detection probe will detach at a different melting temperature dependent upon the presence of a mismatch between the probe and amplicon sequences.

*Statistical analyses.* To compare the FOS and VA-HIT populations, we employed chi-square tests for categorical measures and two-sample t test for continuous measures, using the SYSTAT statistical package.

### Example 4

### Analysis of the associations between each ABC1 polymorphism and plasma lipid profiles in FOS

Analyses were also performed to test for potential associations between each *ABC1* polymorphism and plasma lipid profiles in FOS. Because the VA-HIT subjects were selected primarily on the basis of plasma lipid values and CHD, we did not test for associations in this group, which was, by design, more homogeneous than that of FOS. In FOS, no statistically significant relationships were detected between any of the 3 *ABC1* polymorphisms and plasma lipids (data not shown). This remained the case after adjustment for familial relationships, age, BMI, smoking, alcohol intake, and apolipoprotein E genotype. Although this result is somewhat incongruous with evidence for a role of ABC1 in the regulation of plasma HDL-C levels (9,11-15), we believe that *ABC1* polymorphisms may directly influence intracellular cholesterol trafficking and, ultimately, reverse cholesterol transport. Support for this hypothesis is provided by the results presented in *Example 2* demonstrating that the homozygosity for either the G596A or A2589G polymorphism is associated with decreased cellular cholesterol efflux in healthy, young subjects. Moreover, because HDL-C levels are modulated by other genetic and environmental factors (7, 36), direct effects of *ABC1* polymorphisms on plasma HDL-C concentrations may not be readily apparent, especially in the heterozygous state.

### Methods

*Measurement of plasma lipids and lipoproteins.* Blood samples were collected from subjects, after a 12-14 h fast, into tubes containing 0.1% EDTA. Plasma was isolated and frozen for subsequent analysis of plasma lipid and lipoprotein concentrations. Plasma total cholesterol and triglyceride concentrations were determined using enzymatic assays (37). Plasma HDL-C concentrations were measured after dextran sulfate-magnesium precipitation of apoB-containing lipoproteins (38), and LDL-C levels were calculated with the equation of Friedewald, Levy, and Fredrickson (39).

*Statistical analyses.* To evaluate the relationships between ABC1 genotype and plasma lipids, we used analysis of covariance techniques, employing several different models to adjust for potential confounders. Age, BMI, smoking status, alcohol consumption, and apolipoprotein E genotype were included in these models. Loglinear models were used to examine associations between the 3 polymorphisms and status (FOS or VA-HIT). For all 3 models, statistically significant interactions were detected between ABC1 genotype and status, indicating that differences existed between the FOS and VA-HIT groups with regard to the presence of mutant alleles. Specifically, for each ABC1 polymorphism, those in the FOS group were more likely to be categorized as wild type, whereas VA-HIT subjects were more likely to be classified as heterozygous.

## Claims

1. A polynucleotide comprising a member selected from the group consisting of:
(a) a polynucleotide as set forth in SEQ ID NO:1 with the sequence variants G596A or T1136C or A2589G or G3456C or any combination of these variants;
(b) a polynucleotide fragment of the polynucleotides of (a) containing the variants G596A or T1136C or A2589G or G3456C or any combination of these variants.

2. The polynucleotide of claim 1 wherein the polynucleotide is DNA.

3. A vector containing one or more of the polynucleotides of claim 1 and 2.

4. A host cell containing the vector of claim 3.

5. A process for producing a polypeptide comprising: expressing from the host cell of claim 4 the polypeptide encoded by said DNA.

6. An assay using one of the materials of claims 1 to 4 for the detection of the ABC1 gene transcripts.

7. An assay using one of the materials of claims 1 to 4 for the detection of the ABC1 variants G596A or T1136C or A2589G or G3456C.

8. A polypeptide selected from the group consisting of:
a) a polypeptide having the deduced amino acid sequence of SEQ ID NO:1 and
b) fragments, analogs and derivatives thereof containing the sequence variants of claim 2b.

9. An antibody capable to bind to the polypeptide of claim 8.

10. An assay using one of the materials of claims 8 and 9 for the detection of
(a) the ABC1 protein or fragments, analogs and derivatives thereof,
(b) the deduced protein or fragments, analogs and derivatives thereof from the ABC1 sequence variants G596A or T1136C or A2589G or G3456C .

11. A diagnostic kit for the detection of the ABC1 gene variants G596A or T1 136C or A2589G or G3456C.

12. Modulator of polypeptides encoded by a polynucleotide comprising a member selected from the group consisting of:
(a) a polynucleotide as set forth in SEQ ID NO:1 and
(b) a polynucleotide fragment of the polynucleotide of (a) containing the sequence variants G596A or T1136C or A2589G or G3456C.

13. A pharmaceutical comprising the modulator of claim 12.

14. The modulation of ABC1 transcripts or proteins or fragments thereof by gene therapy.

15. The modulation of ABC1 transcripts or proteins or fragments thereof by antisense or ribozyme technology or RNA decoys.
